# EUROPEAN PATENT SPECIFICATION

(11) **EP 1 047 771 B1**
(45) Date of publication and mention of the grant of the patent: **23.04.2003**
(21) Application number: 99901279.2
(22) Date of filing: 11.01.1999
(51) Int. Cl.: C12N 9/68, C07K 1/113

(54) **METHOD OF REFOLDING ANGIOSTATIN**
VERFAHREN ZUR ANGIOSTATINRÜCKFALTUNG
PROCEDE DE REPLIEMENT DE L'ANGIOSTATINE

(30) Priority: 12.01.1998 US 71247 P
(43) Date of publication of application: 02.11.2000
(73) Proprietor: G.D. SEARLE & CO., Chicago, IL 60680-5110 (US)
(72) Inventor: VIOLAND, Bernard, N., Glencoe, MO 63038 (US); POLAZZI, Joseph, O., Chesterfield, MO 63017-4910 (US); CASPERSON, Gerald, F., Ballwin, MO 63011-2560 (US)
(74) Representative: Weisert, Annekäte, Dipl.-Ing. Dr.-Ing.
(86) International application number: US9900048
(87) International publication number: WO99035248

(56) References cited:
- EP-A- 0 523 296
- WO-A-97/41824
- YIHAI CAO ET AL.: "Kringle domains of human angiostatin" JOURNAL OF BIOLOGICAL CHEMISTRY, vol. 271, no. 46, 15 November 1996 (1996-11-15), pages 29461-29467, XP002086445 MD US cited in the application
- SCOTT CLEARY ET AL.: "Purification and characterization of tissue plasminogen activator kringle-2 domain expressed in Escherichia coli" BIOCHEMISTRY, vol. 28, no. 4, 21 February 1989 (1989-02-21), pages 1884-1891, XP002101498 EASTON, PA US
- BERNHARD FISCHER ET AL.: "Isolation, renaturation, and formation of disulfide bonds of eukaryotic proteins expressed in Escherichia coli as inclusion bodies" BIOTECHNOLOGY AND BIOENGINEERING INCLUDING: SYMPOSIUM BIOTECHNOLOGY IN ENERGY PRODUCTION AND CONSERVATION., vol. 41, no. 1, 5 January 1993 (1993-01-05), pages 3-13, XP000605146 NEW YORK US cited in the application

## Description

An efficient method for purifying and refolding angiostatin in inclusion bodies expressed in bacteria is disclosed. Nucleic acids encoding full-length angiostatin and subfragments encoding kringle domains K1-4, K2-3, K2-4, K1-3, and K1 are also disclosed.

### Angiogenesis

Angiogenesis, the growth of new blood vessels, plays an important role in cancer growth and metastasis. In humans, the extent of vasculature in a tumor has been shown to correlate with the patient prognosis for a variety of cancers (Folkman, J., *Seminars in Medicine of the Beth Israel Hospital, Boston* **333**(26): 1757-1763, 1995; Gasparini. G., *European Journal of* Cancer **32A**(14): 2485-2493, 1996: Pluda. J. M., *Seminars in Oncology* **24**(2): 203-218, 1997). In normal adults, angiogenesis is limited to well controlled situations, such as wound healing and the female reproductive system (Battegay, E.J., *J Mol Med* **73**:-333-346, 1995; Dvorak, H.F., *New Engl J Med,* **315:** 1650-1659, 1986).

Animal studies suggest that tumors can exist in a dormant state, in which tumor growth is limited by a balance between high rates of proliferation and high rates of apoptosis (Holmgren, L. et al., *Nat. Med. (N. Y.)* **1**(2): 149-153, 1995: Hanahan, D. et al., *Cell* **86**(3): 353-364, 1996). The switch to an angiogenic phenotype allows tumor cells to escape from dormancy and to grow rapidly, presumably as the result of a decrease in the apoptotic rate of the tumor cells (Bouck, *Cancer Cells.* **2**(6): 179-185, 1990: Dameron et al., *Cold Spring Harb Symp Quant Biol,* **59:** 483-489, 1994). The control of angiogenesis is thought to be a balance between factors which promote new vessel formation and anti-angiogenic factors with suppress the formation of a neovasculature (Bouck, N. et al., *Advances in Cancer Research* **69:** 135-173. 1996: O'Reilly et al., *Cell (Cambridge, Mass)* **79**(2): 315-328, 1994).

A variety of pro-angiogenic factors have been characterized including basic and acid fibroblast growth factors (bFGF and aFGF) and vascular permeability factor/vascular endothelial growth factor (VPF/VEGF) (Potgens, A. J. G. et al., *Biol. Chem. Hoppe-Seyler* **376**: 57-70, 1995; Ferrara, N., *European Journal of Cancer* **32A**(14): 2413-2442, 1996; Bikfalvi, A. et al., *Endocrine* Reviews **18:** 26-45, 1997). Several endogenous anti-angiogenic factors have also been characterized, including angiostatin (O'Reilly et al., *Cell* **79**(2): 315-328, 1994), endostatin (O'Reilly et al., *Cell* **88**(2): 277-285, 1997), interferon-alpha. (Ezekowitz et al., *N. Engl. J. Med*., *May 28,* **326**(22) 1456-1463, 1992), thrombospondin (Good et al., Proc *Natl Acad Sci USA* **87**(17): 6624-6628, 1990; Tolsma et al., *J Cell Biol* **122**(2): 497-511, 1993), and platelet factor 4 (PF4) (Maione et al., *Science* **247:**(4938): 77-79, 1990).

### Angiostatin

Angiostatin is a 38 kD protein comprising the first three or four kringle domains of plasminogen and was first described in 1994 (O'Reilly, M. S. et al., *Cell* **79**(2): 315-328, 1994). Mice bearing primary (Lewis lung carcinoma-low metastatic) tumors did not respond to angiogenic stimuli such as bFGF in a corneal micropocket assay and the growth of metastatic tumors in these mice was suppressed until the primary tumor was excised. The factor responsible for the inhibition of angiogenesis and tumor growth was designated mouse angiostatin. Angiostatin was also shown to inhibit the growth of endothelial cells in vitro.

Human angiostatin can be prepared by digestion of plasminogen by porcine elastase (O'Reilly et al., *Cell* **79**(2): 315-328, 1994) or with human metalloelastase (Dong et al., *Cell* **88,** 801-810, 1997). The angiostatin produced via porcine elastase digestion inhibited the growth of metastases and primary tumors in mice. O'Reilly et al. *(Cell* **79**(2): 315-328, 1994) demonstrated that human angiostatin inhibited metastasis of Lewis lung carcinoma in SCID mice. The same group (O'Reilly, M. S. et al., *Nat. Med. (N. Y.)* **2**(6): 689-692, 1996) subsequently showed that human angiostatin inhibited the growth of the human tumors PC3 prostate carcinoma, clone A colon carcinoma, and MDA-MB breast carcinoma in SCID mice. Human angiostatin also inhibited the growth of the mouse tumors Lewis lung carcinoma, T241 fibrosarcoma and M5076 reticulum cell carcinoma in C57B1 mice. The precise composition of the molecules is not known because these enzymatically-prepared angiostatins are not well characterized biochemically.

### Expression of angiostatin

Angiostatins of known composition can be prepared by means of recombinant DNA technology and expression in heterologous cell systems. Recombinant human angiostatin comprising Kringle domains one through four (K1-4) has been produced in the yeast *Pichia pastoris* (Sim et al., *Cancer Res* **57**: 1329-1334, 1997). The recombinant human protein inhibited growth of endothelial cells in vitro and inhibited metastasis of Lewis lung carcinoma in C57Bl mice. Recombinant murine angiostatin (K1-4) has been produced in insect cells (Wu et al., *Biochern Biophys* Res *Comm* **236:** 651-654, 1997). The recombinant mouse protein inhibited endothelial cell growth in vitro and growth of primary Lewis lung carcinoma in *vivo.* These experiments demonstrated that the first four kringle domains are sufficient for angiostatin activity but did not determine which kringle domains are necessary.

Cao et al. *(J. Biol. Chem.* **271**: 29461-29467, 1996), produced fragments of human plasminogen by proteolysis and by expression of recombinant proteins in *E. coli.* These authors showed that kringle one and to a lesser extent kringles two and three of plasminogen were responsible for the inhibition of endothelial cell growth in vitro. Specifically, kringles 1-4 and 1-3 inhibited at similar concentrations, while K1 alone inhibited endothelial cell growth at four-fold higher concentrations. Kringles two and three inhibited to a lesser extent. The solubilisation and the refolding of the protein was done at pH 8.0 in the presence of DTT and reduced/oxidised glutathione. According to the invention, a higher pH is used to solubilise and refold the protein. More recently Cao et al. (*J Biol Chem* **272:** 22924-22928, 1997), showed that recombinant mouse or human kringle five inhibited endothelial cell growth at lower concentrations than angiostatin (K1-4). These experiments demonstrated in vitro angiostatin-like activity but did not address in vivo action against tumors and their metastases.

World patent applications WO 95/29242 A1, WO 96/41194 A1, WO 96/35774 A2, and WO 98/54217 A1 describe the expression, purification, and characterization of angiostatin. WO 95/29242 A1 951102 discloses purification of a protein from blood and urine by HPLC that inhibits proliferation of endothelial cells. The protein has a molecular weight between 38 kilodaltons and 45 kilodaltons and an amino acid sequence substantially similar to that of a murine plasminogen fragment beginning at amino acid number 79 of a murine plasminogen molecule. WO 96/41194 A1 961219, discloses compounds and methods for the diagnosis and monitoring of angiogenesis-dependent diseases. WO 96/35774 A2 961114 discloses the structure of protein fragments, generally corresponding to kringle structures occurring within angiostatin. It also discloses aggregate forms of angiostatin, which have endothelial cell inhibiting activity, and provides a means for inhibiting angiogenesis of tumors and for treating angiogenic-mediated diseases. WO 98/54217 A1 describes fragments of angiostatin that have endothelial cell inhibiting activity.

Anti-angiogenic therapy may offer several advantages over conventional chemotherapy for the treatment of cancer. Anti-angiogenic agents have low toxicity in preclinical trials and development of drug resistance has not been observed (Folkman, J., *Seminars in Medicine of the Beth Israel Hospital, Boston* **333**(26): 1757-1763, 1995). As angiogenesis is a complex process, made up of many steps including invasion, proliferation and migration of endothelial cells, it can be anticipated that combination therapies may be most effective. In fact, combinations of chemotherapy with anti-angiogenic therapy have already shown promising results in pre-clinical models (Teicher, B. A. et al., *Breast Cancer Research and Treatment* **36:** 227-236, 1995; Teicher, B. A. et al., *European Journal of Cancer* **32A**(14): 2461-2466, 1996).

### Purification and refolding of angiostatin

Although many types of expression systems have been developed over the past twenty years, bacterial systems, particularly those based on *E. coli,* are widely used for the production of proteins on an industrial scale. Vectors which permit high level expression and the ability to carry out fermentations at high cell densities and low cost, have contributed to the extensive development and use of *E. coli*-based expression systems. One significant problem, however, is the tendency of *E. coli* to form inclusion bodies which contain the desired recombinant protein. Inclusion body formation necessitates additional downstream processing, such as *in vitro* refolding, before biologically active proteins can be recovered. The tendency to form insoluble aggregates does not appear to correlate with factors such as size, hydrophobicity, subunit structure, or the use of fusion domains (Kane J.F. and Harley, D.L., *Tibtech* **6**: 95, 1988). Inclusion body formation also appears to be determined by the rates of protein synthesis, folding, aggregation, and proteolytic degradation, the solubility and thermodynamics of folding intermediates and native proteins, and the interactions of these species with chaperone proteins (Rainer Rudolph, *In* Protein Engineering: Principles and Practice, Edited by Jeffrey L. Cleland and Charles S. Craik, p 283-298, Wiley-Liss, Inc., New York, New York, 1996).

Inclusion bodies generally form in the cytoplasm of cells expressing a recombinant protein at high levels. They refract light when observed by phase contrast microscopy and thus are sometimes referred to as refractile bodies. The inclusion bodies are characterized by a relatively high specific density and can be pelleted from lysed cells by centrifugation. The formation of inclusion bodies may protect recombinant proteins from proteolysis as they do not easily disintegrate under physiological solvent conditions. High concentrations of denaturants, such as 6 M guanidine hydrochloride or 6-8 M urea, have been commonly used to solubilize the proteins present in inclusion bodies. A variety of inclusion body solubilization protocols have been compared (Fisher, B., Summer, L. and Goodenough, P. *Biotechnol. Bioeng.* **1**:3-13, 1992).

Although the desired foreign gene product is the main component of inclusion bodies, other host cell proteins such as small heat shock proteins, outer membrane proteins, elongation factor EF-Tu, and RNA polymerase may also be enriched in such preparations (Allen, S.P., Polazzi, J.O. Gierse, J.K., and Easton, A.M., *J. Bacteriol.* **174:** 6938-6947, 1992); Hart, R.A., Rinas, U., and Bailey, J.E., *J. Biol. Chem.* **265:** 12728-12733, 1990; Hartley, D.L., and Kane, J.F., *Biochem. Soc. Trans.* 16: 101, 1988).

Detailed studies of the refolding of plasminogen-derived kringle domains isolated from bacterial inclusion bodies all suggest that optimal refolding occurs at pH 8.0 (Trexler, M., and Patty, L., *Proc. Natl. Acad. Sci.* **80**: 2457-2461, 1983; Menhart, N., et al., *Biochemistry* **30**: 1948-1957, 1991; Marti, D., et al., *Eur. J. Biochem.,* **219:** 455-462, 1994; Cao, Y., et al., *J. Biol. Chem.* **271:** 29461-29467, 1996). In these studies, inclusion bodies or are generally dissolved in high concentrations of a denaturant (such as urea or guanidine hydrochloride) in the presence of a reducing agent (such as dithiothreitol or beta-mercaptoethanol). In each of these references, the denaturant is diluted either rapidly or slowly to initiate refolding.

World patent application WO 96/35,774 to Folkman et al., filed April 26, 1996, describes proteins derived from human plasminogen which are capable of inhibiting endothelial cell proliferation. Methods for the preparation of aggregate forms of these proteins are also disclosed.

### Summary of the Invention

One object of the invention is to describe a method for expressing high levels of angiostatin and other plasminogen kringles in bacteria.

Another aspect of the invention is to describe an efficient method by which angiostatin inclusion bodies can be solubilized, subsequently refolded, and purified to generate biologically active material.

The steps of solubilizing and refolding angiostatin inclusion bodies are carried out at an elevated pH, ranging from pH 9 to pH 11.5. Preferably the pH range is from pH 10 to pH 11. Even more preferably. the pH is 10.5.

Preferably, the concentration of angiostatin gene product is present at a concentration of from about 1 to about 20 mg/ml during the solubilization step. Even more preferably, the concentration is about 6 mg/ml.

Preferably, the concentration of angiostatin gene product is present at a concentration of from about 0.1 to about 5 mg/ml during the refolding step. Even more preferably, the concentration is about 1 mg/ml.

Preferably a denaturant selected from urea and guanidine hydrochloride is used during the solubilization and refolding steps. Even more preferably, the denaturant is urea.

Preferably the concentration of urea is from about 4 M to about 10 M during the solubilization step. Even more preferably, the concentration is about 6 M.

Preferably the concentration of urea is from about 0.5 M to about 3 M during the refolding step. Even more preferably, the concentration is about 1 M.

Preferably the concentration of guanidine hydrochloride is from about 3 M to about 8 M during the solubilization step. Even more preferably, the concentration is about 5 M.

Preferably the concentration of guanidine hydrochloride is from about 0.2 M to about 2 M during the refolding step. Even more preferably, the concentration is about 0.5 M.

Preferably, the solubilization and reducing steps are carried out in the presence of a reducing agent capable of reducing disulfide linkages to sulfhydryl groups. Preferably the reducing agent is selected from the group consisting of DTT, BME, cysteine, and reduced glutathione. Even more preferably, the reducing agent is DTT.

Preferably, DTT is present at a concentration of from about 2 mM to about 10 mM during the solubilization step. Even more preferably, the concentration is about 6 mM.

Preferably, DTT is present at a concentration of from about 0.5 mM to about 2 mM during the refolding step. Even more preferably, the concentration is about 1 mM.

Preferably, reduced glutathione is present at a concentration of from about 5 mM to about 20 mM during the solubilization step. Even more preferably, the concentration is about 10 mM.

Preferably, reduced glutathione is present at a concentration of from about 1 mM to about 4 mM during the refolding step. Even more preferably, the concentration is about 2 mM.

Preferably, cysteine is present at a concentration of from about 5 mM to about 20 mM during the solubilization step. Even more preferably, the concentration is about 10 mM.

Preferably, cysteine is present at a concentration of from about 1 mM to about 4 mM during the refolding step. Even more preferably, the concentration is about 2 mM.

Preferably, an agent capable of enhancing the interchange of disulfide bonds is present during the refolding step. Preferably the agent is selected from cystine and oxidized glutathione. Even more preferably, the agent is cystine.

Preferably, cystine is present at a concentration of from about 0.2 mM to about 5 mM during the refolding step. Even more preferably, the concentration is about 1 mM.

Preferably the refolding step is carried out in the presence of a ligand capable of binding kringle domains of angiostatin and plasminogen. Preferably the ligand is selected from the group consisting of 6-amino hexanoic acid, 7-amino heptanoic acid, 5-amino pentanoic acid, and 4-amino butyric acid. Even more preferably, the ligand is 6-amino hexanoic acid.

Preferably disulfide bonds are formed through air oxidation during the refolding step. Preferably, the air oxidation step is carried out from about 12 to about 96 hours. Even more preferably, the air oxidation step is carried out from about 24 to about 72 hours. Most preferably the air oxidation step is carried out about 60 hours.

Preferably refolded angiostatin is further purified by a process selected from the group consisting of lysine-sepharose chromatography, ion-exchange chromatography, hydrophobic interaction chromatography and RP-HPLC.

Preferably the method of expression, solubilization, refolding and purification uses an plasminogen-derived kringle domains or angiostatin genes that are human. Even more preferably these genes are selected from the group consisting of SEQ ID NO: 10 through SEQ ID NO: 29.

Preferably the method of expression, solubilization, refolding and purification uses the products of plasminogen-derived kringle domains or angiostatin genes that are human. Even more preferably the products of these genes are encoded by a sequence selected from the group consisting of SEQ ID NO: 10 through SEQ ID NO: 29.

The obtained proteins are a modified human angiostatin amino acid sequence, and said protein can optionally be immediately preceded by (methionine⁻¹), (alanine⁻¹), (methionine⁻², alanine⁻¹), (serine⁻¹), (methionine⁻², serine⁻¹), (cysteine⁻¹), or (methionine⁻², cysteine⁻¹).

Preferably these proteins are selected from the group consisting of angiostatin K1, angiostatin K5, angiostatin K1-K3, angiostatin K1-K4, angiostatin K1-K5.

Additionally, the present invention relates to recombinant expression vectors comprising nucleotide sequences encoding the angiostatin, variants and muteins of angiostatin, related microbial and eukaryotic expression systems, and processes for making (comprising the steps of expressing, solubilizing, refolding, purifying) these proteins.

Cloning of DNA sequences encoding these proteins may be accomplished by the use of intermediate vectors. Alternatively, one gene can be cloned directly into a vector containing the other gene. Linkers and adapters can be used for joining the DNA sequences, as well as replacing lost sequences, where a restriction site was internal to the region of interest. Thus genetic material (DNA) encoding one polypeptide, peptide linker, and the other polypeptide is inserted into a suitable expression vector which is used to transform bacteria, yeast, insect cells or mammalian cells. The transformed organism or cell line is grown and the protein isolated by standard techniques. The resulting product is therefore a new protein which has all or a portion of one protein joined by a linker region to a all or a portion of second protein.

Another aspect of the present invention includes plasmid DNA vectors for use in the expression of these proteins. These vectors contain the novel DNA sequences described above which code for the novel polypeptides of the invention. Appropriate vectors which can transform microorganisms or cell lines capable of expressing the proteins include expression vectors comprising nucleotide sequences coding for the proteins joined to transcriptional and translational regulatory sequences which are selected according to the host cells used.

Vectors incorporating modified sequences as described above are included in the present invention and are useful in the production of the proteins. The vector employed in the method also contains selected regulatory sequences in operative association with the DNA coding sequences of the invention and which are capable of directing the replication and expression thereof in selected host cells.

Methods for producing these proteins is another aspect of the present invention. The method of the present invention involves culturing suitable cells or cell lines, which has been transformed with a vector containing a DNA sequence coding for expression of a novel multi-functional protein. Suitable cells or cell lines may be bacterial cells. For example, the various strains of *E. coli* are well-known as host cells in the field of biotechnology. Examples of such strains include *E. coli* strains JM101 (Yanisch-Perron et al., *Gene* **33:** 103-119, 1985) and MON105 (Obukowicz et al., *Applied Environmental Microbiology* **58:** 1511-1523, 1992). Also included in the present invention is the expression of the multi-functional proteins utilizing a chromosomal expression vector for *E. coli* based on the bacteriophage Mu (Weinberg et al., *Gene* **126:** 25-33, 1993). Various strains of *B. subtilis* may also be employed in this method. Many strains of yeast cells known to those skilled in the art are also available as host cells for expression of the polypeptides of the present invention.

When expressed in the *E. coli* cytoplasm, the gene encoding the proteins of the present invention may also be constructed such that at the 5' end of the gene codons are added to encode Met⁻²-Ala⁻¹, Met⁻²-Ser⁻¹, Met⁻²-Cys⁻¹, or Met⁻¹ at the N-terminus of the protein. The N termini of proteins made in the cytoplasm of *E. coli* are affected by post-translational processing by methionine aminopeptidase (Ben Bassat et al., *J. Bacteriol.* **169:**751-757, 1987) and possibly by other peptidases so that upon expression the methionine is cleaved off the N-terminus. The proteins of the present invention may include polypeptides having Met⁻¹, Ala⁻¹, Ser⁻¹, Cys⁻¹, Met⁻²-Ala⁻¹, Met⁻²-Ser⁻¹, or Met⁻²-Cys⁻¹ at the N-terminus. These mutant proteins may also be expressed in *E. coli* by fusing a secretion signal peptide to the N-terminus. This signal peptide is cleaved from the polypeptide as part of the secretion process.

### Definitions

The following is a list of abbreviations and the corresponding meanings as used interchangeably herein:
BME = beta mercaptoethanol
DTT = dithiothreitol
g = gram(s)
HPLC = high performance liquid chromatography
mg = milligram(s)
ml or mL = milliliter(s)
RT = room temperature
ug or µg = microliter(s)
ul or µl or µL or µL = microliter(s)

The following is a list of definitions of various terms used herein:

The term "anti-tumor" means possessing an activity which slows or abolishes the growth of, or which kills, or otherwise harms tumors *in vivo.*

The term "native sequence" refers to an amino acid or nucleic acid sequence which is identical to a wild-type or native form of a gene or protein.

The terms "mutant amino acid sequence," "mutant protein", "variant protein", "mutein", or "mutant polypeptide" refer to a polypeptide having an amino acid sequence which varies from a native sequence due to amino acid additions, deletions, substitutions, or any combination thereof, or is encoded by a nucleotide sequence from an intentionally-made variant derived from a native sequence or chemically synthesized..

The term "angiostatin" means a protein fragment of plasminogen (encoded by SEQ ID NO: 9) having anti-angiogenic activity. The activity of said fragments can be determined by the mouse corneal micropocket assay of angiogenesis or by inhibition of endothelial cell growth *in vitro.*

The term "kringle" or "K" means a fragment or substructure of plasminogen or angiostatin bounded by disulfide-linked cysteine residues, and which retain a structure similar to that which it will adopt within the whole of plasminogen. A kringle domain may also include interkringle sequences which serve to connect it with other kringle domains. The terms K1, K2, K3, K4, K5 refer to specific kringle domains, and terms like K1-3 or K2-4, for example, refer to contiguous protein segments from the first kringle through the last kringle in a series, including the interkringle sequences.

### Brief description of the figures

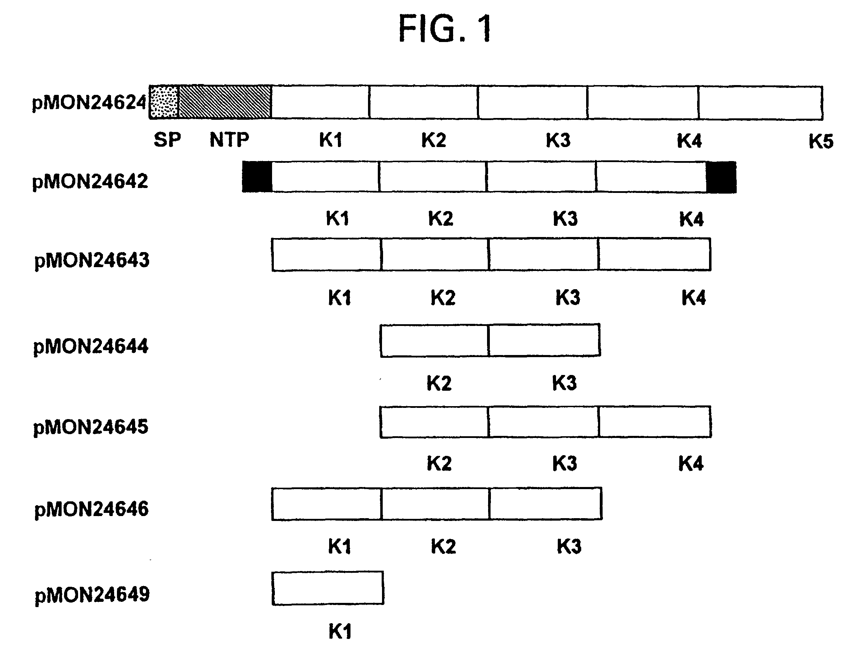

### Figure 1 shows a schematic of selected cloned angiostatin fragments containing kringle domains

The kringle domains of human plasminogen pMON24624 (K1-5), pMON24642 (K1-4E), pMON24643 (K1-4), pMON24644 (2-3), pMON24645 (K2-4), pMON24646 (K1-3), pMON24649 (K1) are shown. The labels K1, K2, K3, K4, and K5, refer to specific kringle domains within human plasminogen or angiostatin. Plasmid pMON24624 contains the sequence of human plasminogen, K1-K5, including the N-terminal peptide (NTP) and the signal peptide (SP). The plasmid pMON24642 encodes kringles K1-K4 plus additional human plasminogen sequences at its 5' and 3' ends shown in the diagram by solid areas. Schematic depictions of the angiostatin-related sequences in plasmids pMON24625 (K1-K4), pMON24626 (K1-K3), pMON24627 (K2-K4), pMON24628 (K2-K3), and pMON24650 (K1) which contain specific kringle domains of human plasminogen are also shown. These plasmids also contain sequences necessary for the expression of the encoded polypeptides in *E. coli.*

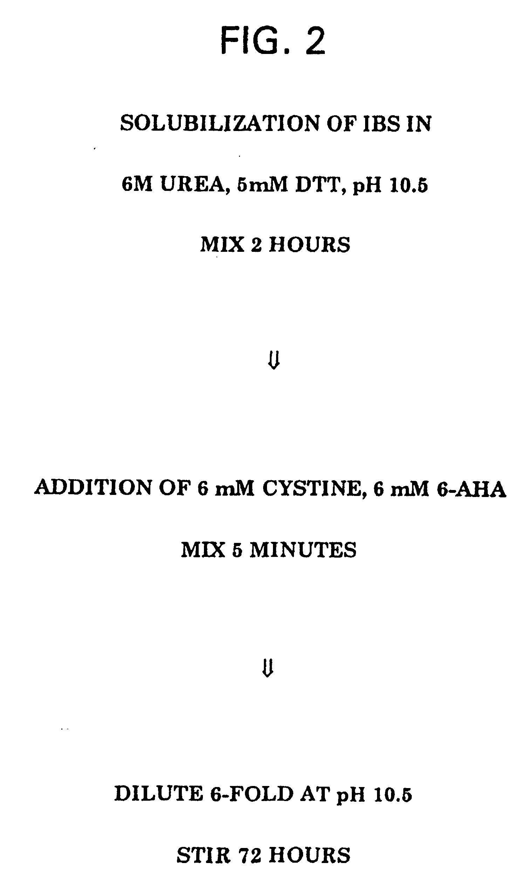

### Figure 2 shows a schematic of the angiostatin refolding procedure

Optimal solubilization conditions in the presence of urea and DTT and refolding in the presence of cystine and 6-amino hexanoic acid are outlined.

### Figure 3 shows the inhibition of HMEC migration by angiostatin

Purified K1-3, K1-4, and elastase-derived angiostatin were assayed in the HMEC cell migration assay at 20 ug/ml. All angiostatins showed inhibition of migration.

### Figure 4 shows the inhibition of BAEC migration by angiostatin

Purified K1-3, K1-4, and elastase-derived angiostatin were assayed in the BAEC cell migration assay at 20 ug/ml. All angiostatins showed inhibition of migration.

### Figure 5 shows the inhibition of HMEC migration by angiostatin

Inhibition of HMEC cell migration versus angiostatin concentration. Dose response curves of angiostatin concentration versus % inhibition of cell migration are shown for K1-3, K1-4, and elastase-derived angiostatin.

### Figure 6 shows binding of refolded angiostatin to lysine-sepharose columns at pH 8.0 and 10.5

Elution profiles for K1-3 at pH 10.5 and single kringle at pH 8.0 by HPLC lysine chromatography are shown.

### Detailed description of the invention

The following examples will illustrate the invention in greater detail, although it will be understood that the invention is not limited to these specific examples.

### General methods

General methods of cloning, expressing, and characterizing proteins are found in T. Maniatis et al., *Molecular Cloning*, *A Laboratory Manual,* Cold Spring Harbor Laboratory, 1982, and references cited therein; and in J. Sambrook et al., *Molecular Cloning, A Laboratory Manual,* 2^{nd} edition, Cold Spring Harbor Laboratory, 1989, and references cited therein.

Unless noted otherwise, all specialty chemicals were obtained from Sigma, Co. (St. Louis, MO). Restriction endonucleases and T4 DNA ligase were obtained from New England Biolabs (Beverly, MA) or Boehringer Mannheim (Indianapolis, IN).

### Strains and plasmids

The bacterial strains used in these studies are listed in Table 1. Plasmids used in or constructed for this study are listed in Table 2.

### Transformation of E. coli strains

*E*. *coli* strains (Table 1), such as DH10B™ (Life Technologies, Gaithersburg, MD) and TG1 (Amersham Corp., Arlington Heights, IL) are used for transformation of ligation reactions and are the hosts used to prepare plasmid DNA for transfecting mammalian cells. *E. coli* strains, such as JM101 (Yanisch-Perron et al., *Gene*, 33: 103-119, 1985) and MON105 (Obukowicz et al., *Appl. and Envir. Micr*., 58: 1511-1523, 1992) can be used for expressing the proteins of the present invention in the cytoplasm or periplasmic space

DH10B™ Subcloning efficiency cells are purchased as competent cells and are ready for transformation using the manufacturer's protocol, while both *E. coli* strains JM101 AND MON105 are rendered competent to take up DNA using a CaCl₂ method. Typically, 20 to 50 mL of cells are grown in LB medium (1% Bacto-tryptone, 0.5% Bacto-yeast extract, 150 mM NaCl) to a density of approximately 1.0 absorbance unit at 600 nanometers (OD600) as measured by a Baush & Lomb Spectronic spectrophotometer (Rochester, NY). The cells are collected by centrifugation and resuspended in one-fifth culture volume of CaCl₂ solution [50 mM CaCl₂, 10 mM Tris-Cl ((10 mM 2-amino-2-(hydroxymethyl) 1,3-propanediol hydrochloride, pH7.4] and are held at 4°C for 30 minutes. The cells are again collected by centrifugation and resuspended in one-tenth culture volume of CaCl₂ solution. Ligated DNA is added to 0.2 mL of these cells, and the samples are held at 4°C for 30-60 minutes. The samples are shifted to 42°C for two minutes and 1.0 mL of LB is added prior to shaking the samples at 37°C for one hour. Cells from these samples are spread on plates (LB medium plus 1.5% Bacto-agar) containing either ampicillin (100 micrograms/mL, ug/mL) when selecting for ampicillin-resistant transformants, or spectinomycin (75 ug/mL) when selecting for spectinomycin-resistant transformants. The plates are incubated overnight at 37°C.

Colonies are picked and inoculated into LB plus appropriate antibiotic (100 ug/mL ampicillin or 75 ug/mL spectinomycin) and are grown at 37°C while shaking.

### DNA isolation and characterization

Plasmid DNA can be isolated by a number of different methods and using commercially available kits known to those skilled in the art. Plasmid DNA is isolated using the Promega Wizard™ Miniprep kit (Madison, WI), the Qiagen QIAwell Plasmid isolation kits (Chatsworth, CA) or Qiagen Plasmid Midi or Mini kit. These kits follow the same general procedure for plasmid DNA isolation. Briefly, cells are pelleted by centrifugation (5000 x g), the plasmid DNA released with sequential NaOH/acid treatment, and cellular debris is removed by centrifugation (10000 x g). The supernatant (containing the plasmid DNA) is loaded onto a column containing a DNA-binding resin, the column is washed, and plasmid DNA eluted. After screening for the colonies with the plasmid of interest, the *E. coli* cells are inoculated into 50-100 ml of LB plus appropriate antibiotic for overnight growth at 37°C in an air incubator while shaking. The purified plasmid DNA is used for DNA sequencing, further restriction enzyme digestion, additional subcloning of DNA fragments and transfection into *E. coli,* mammalian cells, or other cell types.

### Sequence confirmation

Purified plasmid DNA is resuspended in deionized H₂O and its concentration is determined by measuring the absorbance at 260/280 nm in a Bausch and Lomb Spectronic 601 UV spectrometer. DNA samples are sequenced using ABI PRISM™ DyeDeoxy™ terminator sequencing chemistry (Applied Biosystems Division of Perkin Elmer Corporation, Lincoln City, CA) kits (Part Number 401388 or 402078) according to the manufacturer's suggested protocol usually modified by the addition of 5% DMSO to the sequencing mixture. Sequencing reactions are performed in a DNA thermal cycler (Perkin Elmer Corporation, Norwalk, CT) following the recommended amplification conditions. Samples are purified to remove excess dye terminators with Centri-Sep™ spin columns (Princeton Separations, Adelphia, NJ) and lyophilized. Fluorescent dye labeled sequencing reactions are resuspended in deionized formamide, and sequenced on denaturing 4.75% polyacrylamide-8M urea gels using ABI Model 373A and Model 377 automated DNA sequencers. Overlapping DNA sequence fragments are analyzed and assembled into master DNA contigs using Sequencher DNA analysis software (Gene Codes Corporation, Ann Arbor, MI).

### Small-scale expression of angiostatin in E. coli

*E. coli* strain MON105 or JM101 harboring the plasmid of interest is grown at 37°C in M9 plus casamino acids medium with shaking in an air incubator Model G25 from New Brunswick Scientific (Edison, NJ). Growth is monitored at OD₆₀₀ until it reaches a value of 1.0 at which time nalidixic acid (10 mg/mL) in 0.1 N NaOH is added to a final concentration of 50 µg/mL. The cultures are then shaken at 37°C for three to four additional hours. A high degree of aeration is maintained throughout THE culture period in order to achieve maximal production of the desired gene product. The cells are examined under a light microscope for the presence of inclusion bodies (IB). One mL aliquots of the culture are removed for analysis of protein content by boiling the pelleted cells. treating them with reducing buffer and electrophoresis via SDS-PAGE (see Maniatis et al., "Molecular Cloning: A Laboratory Manual", 1982). The culture is centrifuged (5000 x g) to pellet the cells.

### Large (10L)-scale expression of angiostatin in E. coli

The Angiostatin molecules were all scaled up in a 10 L Biostat E™ fermenter (B. Braun Biotech Inc., Allentown PA). The fermentation medium used was M9 salts supplemented with 2% casamino acids (Difco Laboratories, Detroit MI) and glucose. Approximately one milliliter of a thawed culture was transferred to a 3.8 L Fernbach shake flask containing 1.0 L of medium and then incubated at 37°C at a shaker speed of 250 rpm for 12 hours. This shake flask culture was then used to inoculate a 10 L fermenter containing 9.0 L of medium. The fermentation conditions were as follows: agitation = 1000 rpm, sparger airflow rate = 15 liters/min, pH control = 7.0 by the addition of NH₄OH, backpressure = 10 psi, dissolved oxygen control > 30%, and temperature = 37°C. Glucose was initially batched in at 15 g/l and controlled at 2-5 g/l by the addition of a 50% glucose feed stock. The fermentation culture was grown to an initial OD (550nm) = 12-15 and induced with 50 mg/l nalidixic acid. The fermentation was harvested four hours post induction by continuous-flow centrifugation.

### 10L scale inclusion body isolation

The cell paste from a 10L fermentation was resuspended in approximately 6.0 L of 50 mM Tris/150 mM EDTA buffer containing 40 pellets of 50 ml Complete Protease Inhibitor Cocktail (Boehringer Mannheim; Indianapolis, IN) and 600,000 KIU of Trasyol. The resuspension was passed once through a Microfluidizer (Newton, MA) at 10,000 psi and temperature maintained under 8°C. The recovered homogenate was then spun at 15,000 x G for 25 minutes and mixed to consistency with an Ultr-Turrax mixer. The cell paste resuspension was then homogenized by passing the cell suspension two times through a Microfluidizer (Newton, MA) operating at a pressure of 10,000 psi. The temperature of the homogenate was maintained less than 6°C by collection in a stainless steel container placed in an ice bath. Inclusion bodies were then isolated by centrifugation of the cell homogenate at 15,000 X g for 30 minutes and removal of supernatant. Inclusion bodies were then washed a total of two times by resuspending the inclusion bodies in chilled D.I. and centrifugation at 15,000 X g for 30 minutes. Angiostatin Inclusion bodies were then frozen at -70°C.

### Small-scale isolation of inclusion bodies

The cell pellet from a 330 mL *E. coli* culture is resuspended in 15 mL of sonication buffer Tris-Cl, pH 8.0 + 1 mM ethylenediaminetetraacetic acid (EDTA). These resuspended cells are sonicated using the microtip probe of a Sonicator Cell Disruptor (Model W-375, Heat Systems-Ultrasonics, Inc., Farmingdale, New York). Three rounds of sonication in sonication buffer followed by centrifugation are employed to disrupt the cells and wash the inclusion bodies (IB). The first round of sonication is a 3 minute burst followed by a 1 minute burst, and the final two rounds of sonication are for 1 minute each.

### Extraction and refolding of proteins from inclusion body pellets

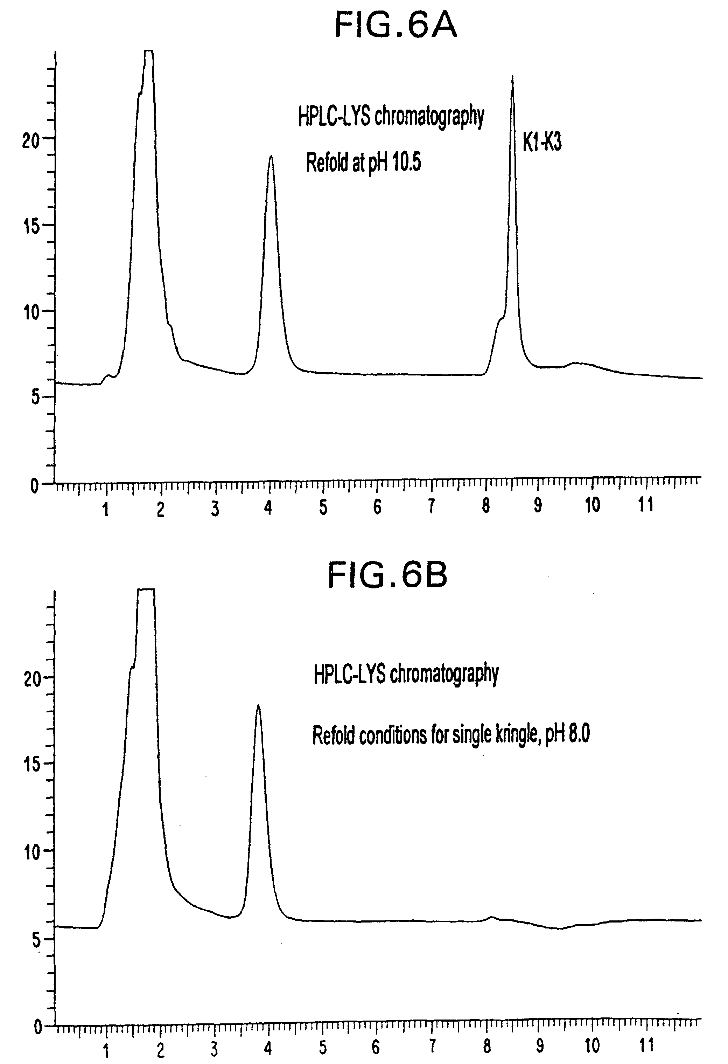

All steps are at 4°C. Angiostatin K1-4 inclusion bodies were dissolved in 6M urea, 5 mM DTT, 50 mM Bis-Tris Propane, pH 10.8 at 1.2 mg/ml. This solution was stirred for 2 hours and then 6-amino hexanoic acid (stock 1M) was added to achieve a 6 mM concentration, followed by addition of cystine (stock 0.2M, pH 10.5) to 6 mM. This was mixed for 5 minutes and then the K1-4 angiostatin was diluted to 0.2 mg/ml by addition of 75 mM Bis-Tris propane, pH 10.5. It was then stirred for 72 hours to complete the refolding of the protein as assessed by reverse-phase HPLC or by binding to Lys-HPLC (Figure 6).

### Purification

Purification of angiostatin K1-4 was achieved using a Sepharose-Lys affinity column followed by Q-Sepharose chromatography. The sample was next dialyzed against 50 mM sodium phosphate, pH 8.0 and then filtered through a 1 uM filter to clarify. This sample was loaded onto a Sepharose-Lys column equilibrated in 50 mM sodium phosphate, pH 8.0. The load was at approximately 1.5 gm of K1-4 per ml of resin. After loading the column, it was washed with 1 column volume of equilibrating buffer and then K1-4 was eluted using a 10 column volume gradient from 0-8 mM 6-amino hexanoic acid in 50 mM sodium phosphate, pH 8.0.

Fractions were analyzed by SDS-gel electrophoresis and/or RP-HPLC and pooled. This pool was made 40 mM Tris-Cl, and adjusted to pH 9.0. It was then dialyzed extensively vs. 40 mM Tris-Cl, pH 9.0 and applied to a Pharmacia Q-Sepharose HP column (∼5 mgs protein/ml resin) equilibrated in the same buffer. K1-4 was then eluted using a NaCl gradient from 0-0.1M in the equilibrating buffer. Fractions were analyzed by RP-HPLC and/or SDS gel electrophoresis and pooled.

In some cases the folded proteins can be affinity-purified using affinity reagents such as monoclonal antibodies or receptor subunits attached to a suitable matrix. Purification can also be accomplished using any of a variety of chromatographic methods such as: ion exchange, gel filtration or hydrophobic interaction chromatography or reversed phase HPLC. These and other protein purification methods are described in detail in Methods in Enzymology, Volume 182 "Guide to Protein Purification" edited by Murray Deutscher, Academic Press, San Diego, California, 1990.

### Protein characterization

The purified protein is analyzed by RP-HPLC, electrospray mass spectrometry, and SDS-PAGE. The protein quantitation is done by amino acid composition, RP-HPLC, and Bradford protein determination. In some cases tryptic peptide mapping is performed in conjunction with electrospray mass spectrometry to confirm the identity of the protein.

### Inhibitory effect on proliferation of Daudi cells

Human Burkitt's lymphoma Daudi cells (ATCC) are seeded at 2 x 10⁴ cells/well into 96-well tissue culture plates and cells are cultured in the presence or absence of serial doses of rhIFN.alpha. 2b for 3 days. Cultures are pulsed with ³H-thymidine for the last hour of the culture period, and the ³H-thymidine uptake, counts per minute (cpm), ARE measured on a Beta-plate reader. All samples are assayed in triplicate.

### Endothelial cell proliferation assay

The endothelial cell proliferation assay was performed as described by Cao et al. *(J. Biol. Chem.* **271:** 29461-29467, 1996). Briefly, human dermal microvascular endothelial cells (HdMVEC, Clonetics) or bovine microvascular endothelial cells (BacEnd, Incell) were maintained in MCDB131 containing 5% heat-inactivated fetal bovine serum (FBS, Hyclone), antibiotics, 100 ug/ml heparin (Sigma) and 100 ug/ml endothelial mitogen (Biomedical Technologies). Confluent monolayers at passages 2-5 were dispersed in 0.05% trypsin and resuspended in complete medium. Five hundred ul of complete media containing 1.25 x 10⁴ cells were seeded into wells of a 24-well tissue culture plate coated with 0.1% gelatin (Sigma). The cells were incubated overnight at 37°C/5% CO₂ at which time the media was replaced with 250 ul of media containing 5% FBS and various concentrations of inhibitors. After 30 minutes of incubation, 250 ul of media containing 1 ng/ml bFGF (R&D Systems) was added and the cells were incubated for an additional 72 hours, at which time they were trypsinized and counted with a Coulter counter.

### Endothelial cell in vitro tube formation assay

The tube formation assay is performed as previously described (Gately et al., *Cancer Res*. **56**:4887-4890, 1996). The effect of angiogenesis inhibitors can be tested in an *in vitro* angiogenesis assay; endothelial cell tube formation on matrix. Matrigel (Becton Dickinson) was thawed on ice and diluted 1:1 with MCDB 131 medium supplemented with 2 mM L-glutamine, containing either buffer control or testing compounds. Aliquots of 0.5 ml of 1:1 diluted matrigel were plated into individual wells of 2-chambered Tissue-Tek polystyrene slides (Nunc) and allowed to polymerize at 37°C for at least 30 min. Primary bovine capillary endothelial cells derived from the bovine adrenal cortex (purchased from Incell) were cultured (as described for bovine endothelial cell proliferation). Following trypsinization, the cells were washed three times, then resuspended at 3.5 x 10⁵ cells/ml in MCDB131 medium supplemented with L-glutamine and 1% fetal bovine serum containing either the same buffer control or testing compounds. The endothelial cells (0.5 ml at 3.5 x 10⁵ cells /ml) were plated onto matrigel coated wells containing the same additives. After incubation for 16-24 hours at 37°C in a 5% CO2 incubator, cultures were evaluated for tube formation (sprouting). Random fields of each experimental condition were photographed using a Nikon N6006 camera under a Nikon Diaphot microscope. The photographs were then quantitated by measuring the total length of the tubes. Tube formation can also be quantitated using image analysis software. Endothelial cell tube networks were fixed in 100% methanol at -20°C for 7-10 min, rinsed 4 times with phosphate buffered saline (PBS) and incubated overnight at 4°C with rabbit polyclonal anti-human Factor VIII-related antigen (von Willibrand factor, vWF) (Dako). The next day, the cells were stained with a secondary antibody, Cy3-conjugated goat anti-rabbit IgG F(ab')2 specific (Jackson ImmunoResearch Laboratories). Tube formation was visualized using a Nikon microphot-FXA with a fluorescence filter linked to a computer containing image analysis software. Tube formation was measured as follows: Total area encompassed by endothelial cell tubes/Total endothelial cell surface area.

### Endothelial cell migration assay

The endothelial cell migration assay is performed essentially as previously described (Gately et al., *Cancer Res.* **56:**4887-4890, 1996). To determine the ability of angiostatin to inhibit the migration of endothelial cells, migration assays were performed in a transwell chamber (Costar) containing 8 mm pore size polycarbonate membranes. The cells utilized in the assay were either human microvascular endothelial cells from Emory or bovine endothelial cells (kindly provided by Gately Northwestern University, Evanston, IL). The cells were starved overnight before use in MCDB131 + 0.1% BSA (human cells) or DMEM+0.1%BSA (bovine cells), harvested, and resuspended in the same media at 10⁶ cells/ml. The lower side of the transwells were coated with 0.1% gelatin for 30 minutes at 37°C before addition of 2 x 10⁵ cells to the upper chamber. The transwell was moved to a well containing the chemoattractant (bFGF or VEGF) in the lower chamber. Migration was allowed to occur overnight at 37°C. The membranes were then fixed and stained, and the number of cells that migrated to the lower side of the membrane counted in 3 high powered fields.

### Example 1: Construction of pMON24642 and selection of strains producing high levels of angiostatin K1-4E

A cDNA encoding angiostatin K1-4E (see e.g., pMON24642-a12.seq, SEQID # 10) was synthesized by polymerase chain reaction using the oligonucleotide primers angec-s1 (angec-sl.seq, SEQID # 1) and angec-n1 (angec-n1.seq, SEQID # 4) and using the human plasminogen cDNA (pMON24624.seq SEQID # 9) as template. The resulting DNA was cleaved with restriction endonucleases *Afl*III and *Hin*dIII and was ligated into the *E. coli* expression vector pMON5723 (Olins and Rangwala, *Methods Enzymol.* **185** (Gene Expression Technol.): 115-119, 1990) which had been digested with *Nco*I and *Hin*dIII. A portion of the ligation reaction was used to transform *E. coli* strain DH10B to spectinomycin resistance.

Plasmid DNA was isolated from the DH10B bacterial colonies and used to transform *E. coli* strain MON105 to spectinomycin resistance. Individual antibiotic resistant colonies were inoculated into 1 ml of Luria Broth with 75 ug/ml spectinomycin and grown over night at 37 degrees with shaking. Fifty microliters of the overnight culture were inoculated into one ml of M9 medium with casamino acids and grown for four hours at 37 degrees with shaking. Nalidixic acid (50 ul of a 1 mg/ml aqueous solution) was added and the cultures were incubated at 37 degrees for an additional three hours. Cultures were examined by phase-contrast microscopy for the presence of cytoplasmic inclusion bodies and by SDS polyacrylamide gel electrophoresis for the presence of the expressed protein. Three cultures expressing the most angiostatin were selected for further characterization. Plasmid DNA was isolated from clones A12, B5, and B6 and was analyzed by DNA sequencing. The sequences of the cDNAs in these plasmids are shown in 24642-A12.seq, 24642-B5.seq and 24642-B6.seq (SEQID # 10, SEQID # 11, and SEQID # 12, respectively).Each cDNA encoded the expected protein but contained various silent mutations at the third positions of codons two through eight. These mutations were caused by the redundancies in the 5' PCR primer (angec-s1.seq, SEQID # 1), and the particular mutations in these clones result in high level expression of angiostatin. Each cDNA differed from the others and from the wild-type sequence at one or more of these positions.

### Example 2: Construction of pMON24643 and selection of strains producing high levels of angiostatin K1-4

A cDNA encoding angiostatin K1-4 was synthesized by polymerase chain reaction using the oligonucleotide primers angec-s2 (angec-s2.seq, SEQID # 2) and angec-n2 (angec-n2.seq, SEQID # 5) and using the human plasminogen cDNA (pMON24624.seq, SEQID # 9) as template. The resulting cDNA was cleaved with restriction endonucleases *Afl*III and *Hin*dIII and was ligated into the *E. coli* expression vector pMON5723 which had been digested with *Nco*I and *Hin*dIII. A portion of the ligation reaction was used to transform *E. coli* strain DH10B to spectinomycin resistance

Plasmid DNA was isolated from the DH10B bacterial colonies and used to transform *E. coli* strain MON105 to spectinomycin resistance. Individual antibiotic resistant colonies were inoculated into 1 ml of Luria Broth with 75 ug/ml spectinomycin and grown over night at 37 degrees with shaking. Fifty microliters of the overnight culture were inoculated into one ml of M9 medium with casamino acids and grown for four hours at 37 degrees with shaking. Nalidixic acid (50 ul of a 1 mg/ml aqueous solution) was added and the cultures were incubated at 37 degrees for an additional three hours. Cultures were examined by phase-contrast microscopy for the presence of cytoplasmic inclusion bodies and by SDS polyacrylamide gel electrophoresis for the presence of the expressed protein. Three cultures expressing the most angiostatin were selected for further characterization. Plasmid DNA was isolated from clones C10, D5, and D7 and was analyzed by DNA sequencing. The sequences of the cDNAs in these plasmids are shown in 24643-C10.seq, 24643-D5.seq and 24643-D7.seq (SEQID # 13, SEQID # 14, and SEQID # 15, respectively). Each cDNA encoded the expected protein but contained various silent mutations at the third positions of codons two through eight. These mutations were caused by the redundancies in the 5' PCR primer (angec-s2.seq, SEQID # 2), and the particular mutations in these clones result in high level expression of angiostatin. Each cDNA differed from the others and from the wild-type sequence at one or more of these positions.

### Example 3: Construction of pMON24644 and selection of strains producing high levels of angiostatin K2-3

A cDNA encoding angiostatin K2-3 was synthesized by polymerase chain reaction using the oligonucleotide primers angec-s3 (angec-s3.seq, SEQID # 3) and angec-n3 (angec-n3.seq, SEQID # 6) and using the human plasminogen cDNA (pMON24624.seq, SEQID # 9) as template. The resulting cDNA was cleaved with restriction endonuclease *Afl*III and *Hin*dIII and was ligated into the *E. coli* expression vector pMON5723 which had been digested with *Nco*I and *Hin*dIII. A portion of the ligation reaction was used to transform *E. coli* strain DH10B to spectinomycin resistance

Plasmid DNA was isolated from the DH10B bacterial colonies used to transform *E. coli* strain MON105 to spectinomycin resistance. Individual antibiotic resistant colonies were inoculated into 1 ml of Luria Broth with 75 ug/ml spectinomycin and grown over night at 37 degrees with shaking. Fifty microliters of the overnight culture were inoculated into one ml of M9 medium with casamino acids and grown for four hours at 37 degrees with shaking. Nalidixic acid (50 ul of a 1 mg/ml aqueous solution) was added and the cultures were incubated at 37 degrees for an additional three hours. Cultures were examined by phase-contrast microscopy for the presence of cytoplasmic inclusion bodies and by SDS polyacrylamide gel electrophoresis for the presence of the expressed protein. Three cultures expressing the most angiostatin were selected for further characterization. Plasmid DNA was isolated from clones E7, E9, and F8, and was analyzed by DNA sequencing. The sequences of the cDNAs in these plasmids are shown in 24644-E7.seq, 24644-E9.seq and 24644-F8.seq (SEQID # 16, SEQID # 17, and SEQID # 18, respectively). Each cDNA encoded the expected protein but also contained mutations which altered one or more amino acids. Each cDNA also contained various silent mutations at the third positions of codons two through eight. These mutations were caused by the redundancies in the 5' PCR primer (angec-s3.seq, SEQID # 3), and the particular mutations in these clones result in high level expression of angiostatin. Each cDNA differed from the others and from the wild-type sequence at one or more of these positions.

### Example 4: Construction of pMON24645 and selection of strains producing high levels of angiostatin K2-4

A cDNA encoding angiostatin K2-4 was synthesized by polymerase chain reaction using the oligonucleotide primers angec-s3 (angec-s3.seq, SEQID # 3) and angec-n2 (angec-n2.seq, SEQID # 5) and using the human plasminogen cDNA (pMON24624.seq, SEQID # 9) as template. The resulting cDNA was cleaved with restriction endonuclease *Afl*III and *Hind*III and was ligated into the *E. coli* expression vector pMON5723 which had been digested with *Nco*I and *Hind*III. A portion of the ligation reaction was used to transform *E. coli* strain DH10B to spectinomycin resistance

Plasmid DNA was isolated from the DH10B bacterial colonies and used to transform *E. coli* strain MON105 to spectinomycin resistance. Individual antibiotic resistant colonies were inoculated into 1 ml of Luria Broth with 75 ug/ml spectinomycin and grown over night at 37 degrees with shaking. Fifty microliters of the overnight culture were inoculated into one ml of M9 medium with casamino acids and grown for four hours at 37 degrees with shaking. Nalidixic acid (50 ul of a 1 mg/ml aqueous solution) was added and the cultures were incubated at 37 degrees for an additional three hours. Cultures were examined by phase-contrast microscopy for the presence of cytoplasmic inclusion bodies and by SDS polyacrylamide gel electrophoresis for the presence of the expressed protein. Three cultures expressing the most angiostatin were selected for further characterization. Plasmid DNA was isolated from clones G3, H5, and H6, and was analyzed by DNA sequencing. The sequences of the cDNAs in these plasmids are shown in 24645-G3.seq, 24645-H5.seq and 24645-H6.seq (SEQID # 19, SEQID # 20, and SEQID # 21, respectively). Each cDNA encoded the expected protein but clone H5 contained a mutation which altered one amino acid. Each cDNA also contained various silent mutations at the third positions of codons two through eight. These mutations were caused by the redundancies in the 5' PCR primer (angec-s3.seq, SEQID # 3), and the particular mutations in these clones result in high level expression of angiostatin. Each cDNA differed from the others and from the wild-type sequence at one or more of these positions.

### Example 5: Construction of pMON24646 and selection of strains producing high levels of angiostatin K1-3

A cDNA encoding angiostatin K1-3 was synthesized by polymerase chain reaction using the oligonucleotide primers angec-s2 (angec-s2.seq, SEQID # 2) and angec-n3 (angec-n3.seq, SEQID # 6) and using the human plasminogen cDNA (pMON24624.seq, SEQID # 9) as template. The resulting cDNA was cleaved with restriction endonuclease *Afl*III and *Hin*dIII and was ligated into the E. *coli* expression vector pMON5723 which had been digested with *Nco*I and *Hin*dIII. A portion of the ligation reaction was used to transform *E. coli* strain DH10B to spectinomycin resistance.

Plasmid DNA was isolated from the DH10B bacterial colonies and used to transform *E. coli* strain MON105 to spectinomycin resistance. Individual antibiotic resistant colonies were inoculated into 1 ml of Luria Broth with 75 ug/ml spectinomycin and grown over night at 37 degrees with shaking. Fifty microliters of the overnight culture were inoculated into one ml of M9 medium with casamino acids and grown for four hours at 37 degrees with shaking. Nalidixic acid (50 ul of a 1 mg/ml aqueous solution) was added and the cultures were incubated at 37 degrees for an additional three hours. Cultures were examined by phase-contrast microscopy for the presence of cytoplasmic inclusion bodies and by SDS polyacrylamide gel electrophoresis for the presence of the expressed protein. Six cultures expressing the most angiostatin were selected for further characterization. Plasmid DNA was isolated from clones A7, A9, A10, A12, B1, and B10, and was analyzed by DNA sequencing. The sequences of the cDNAs in these plasmids are shown in 24646-A7.seq, 24646-A9.seq and 24646-A10, 24646-A12, 24646-B1, and 24646-B10 (SEQID # 22, SEQID # 23, SEQID # 24, SEQID # 25, SEQID # 26, and SEQID # 27, respectively). Each cDNA encoded the expected protein. Each cDNA also contained various silent mutations at the third positions of codons two through eight. These mutations were caused by the redundancies in the 5' PCR primer (angec-s2,seq, SEQID # 2), and the particular mutations in these clones result in high level expression of angiostatin. Each cDNA differed from the others and from the wild-type sequence at one or more of these positions.

### Example 6: Construction of pMON24649 and of strains producing high levels of angiostatin K1

A cDNA encoding angiostatin K1 (24649.seq, SEQID # 28) was synthesized by polymerase chain reaction using the oligonucleotide primers angec-sk1 (angec-sk1.seq, SEQID # 7) and angec-n4 (angec-n4.seq, SEQID # 8) and using the cDNA in pMON24646-A7 (SEQID # 22) as template. The 5' PCR primer (angec-sk1) contained the same silent mutations in the first eight codons that resulted in high level expression of angiostatin K1-3 from pMON24646-A7. The resulting cDNA was cleaved with restriction endonucleases *Nco*I and *Hin*dIII and was ligated into the *E. coli* expression vector pMON5723 which had also been digested with *Nco*I and *Hin*dIII. A portion of the ligation reaction was used to transform *E. coli* strain DH10B to spectinomycin resistance. Plasmid DNA was isolated from the bacterial colonies and analyzed by restriction endonuclease cleavage and DNA sequencing.

Plasmid DNA was then used to transform *E. coli* strain MON105 to spectinomycin resistance. Individual colonies were grown over night in Luria Broth containing 75 ug/ml spectinomycin. One ml of the over night culture was used to inoculate 20 ml of M9 medium containing casamino acids. After about three hours of growth at 37 degrees, Nalidixic acid was added and the cultures incubated for a further three hours at 37 degrees. Cultures were screened by phase contrast microscopy for the presence of cytoplasmic inclusion bodies and by SDS polyacrylamide gel electrophoresis for expression of the protein. Unlike other forms of angiostatin, K1 protein was not incorporated into inclusion bodies, but rather remained soluble in the cytoplasm of the bacteria. Nevertheless, the mutations in the first eight codons in pMON24649 conferred high level expression of K1 as they had for K1-3 expression from pMON13646-A7.

### Example 7: Method for refolding angiostatin

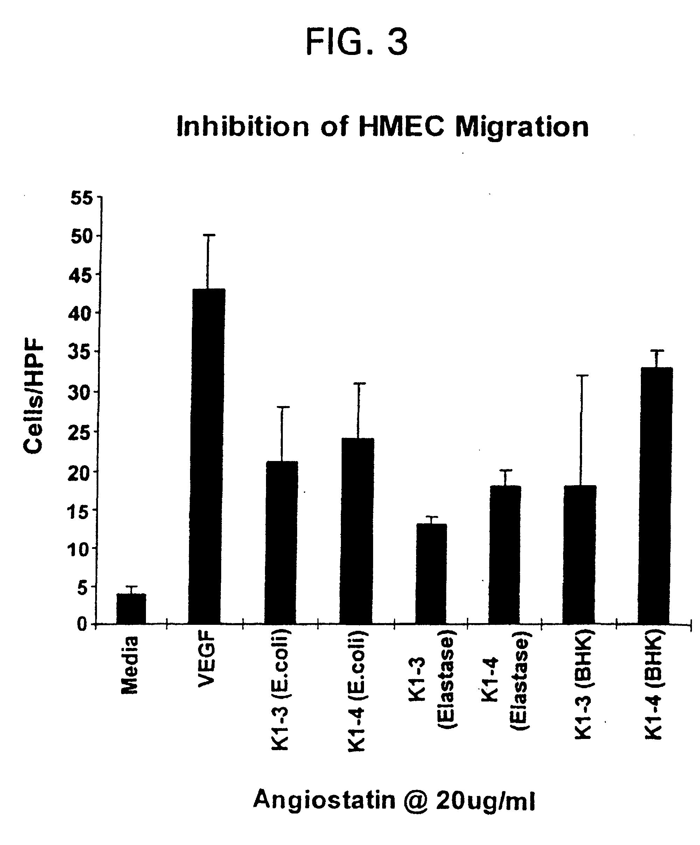

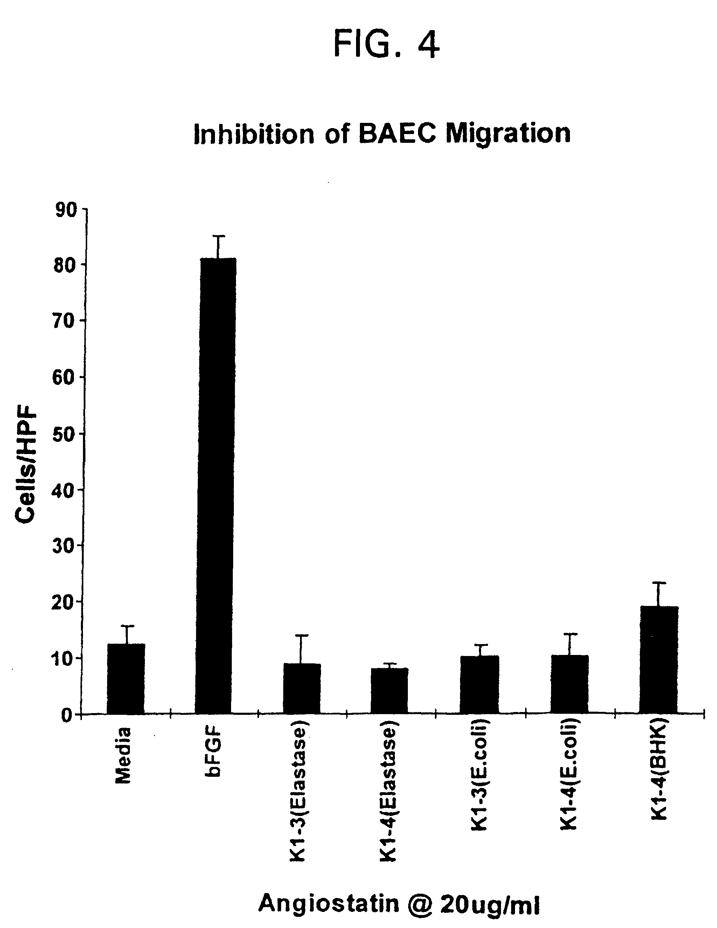

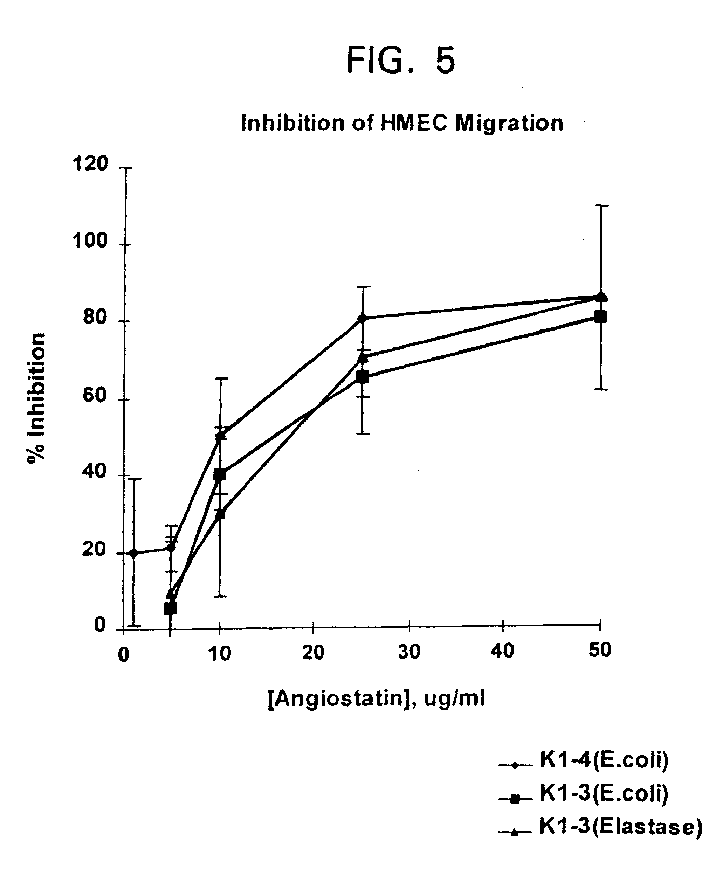

All steps are at 4°C. Angiostatin K1-4 inclusion bodies were dissolved in 6M urea, 5 mM DTT, 50 mM Bis-Tris Propane, pH 10.8 at 1.2 mg/ml. This solution was stirred for 2 hours. A stock solution of 1M 6-amino hexanoic acid was then added to achieve a 6 mM concentration, followed by addition of cystine (stock 0.2M, pH 10.5) to 6 mM. This was stirred for 5 minutes. The K1-4 angiostatin was then diluted to 0.2 mg/ml by addition of 75 mM Bis-Tris propane, pH 10.5. It was then stirred for 72 hours to complete the refolding of the protein. Refolded angiostatin was purified by lysine-Sepharose chromatography and Q-Sepharose-HP chromatography. This purified angiostatin was assayed for inhibition of cell migration as shown in figures 3-5.

### Tables

**Table 1:**

| ***Strains*** | | |
|---|---|---|
| **Designation** | **Description or Genotype** | **Referenee/Source** |
| DH10B | F⁻ *mcr*A *Δ*(*mrr-hsd*RMS-*mcr*BC) φ*dlac*ZΔM15 *Δlac*X74 *deo*R *rec*A1 *end*A1 *ara*D139 Δ(ara, leu)7697 *gal*U *gal*K λ·*rps*L *nup*G | Life Technologies, Rockville, Maryland |
| | | |
| MON105 (ATCC # 55204) | F⁻, lambda-,IN (rrnD, rrnE)1, rpoD⁺, *rpo*H358 | Obukowicz et al., *Appl. and Envir. Micr.,* **58:** 1511-1523, **1992** |
| | | |
| JM101 (ATCC # 33876) | delta (*pro lac*), *sup*E, *thi,* F'(*tra*D36, *pro*A⁺B⁺, *lac*I^{q}, *lac*ZdeltaM15) | Yanisch-Perron et al., *Gene,* **33:** 103-119, 1985 |

**Table 2:**

| ***Plasmids*** | | | | |
|---|---|---|---|---|
| **Plasmid** | **SEQ ID NO.** | **Marker** | **Description** | **Source** |
| pMON5723 | | Spec | Generic Spec-resistant *E. coli* expression vector containing the *E. coli rec*A1 promoter and G10L ribosome binding site | (Olins and Rangwala, *Methods Enzymol.* **185** (Gene Expression Technol.): 115-119, 1990) |
| | | | | |
| pMON6875 | | Spec | Generic Spec-resistant *E. coli* expression vector containing the *E. coli rec*A1 promoter and G10L ribosome binding site | (Olins and Rangwala, *Methods Enzymol.* **185** (Gene Expression Technol.): 115-119, 1990) |
| | | | | |
| pMON24624 | #9 | Amp | Human plasminogen, including the native plasminogen signal sequence, N-terminal peptide and kringles 1-5. *Bam*HI fragment in pMON3360B. (Amino acids -19-537 of plasminogen, nucleotides 55-1752) | This work |
| | | | | |
| pMON24642-A12, -B5, -B6 | #10, #11, #12 | spec | Human angiostatin(E). *Afl*III/*Hind*III fragment in pMON5723. The 5' end has been mutagenized for increased expression. (Amino acids 74-470 of plasminogen nucleotides 331-1464) | This work |
| | | | | |
| pMON24643-C10, D5,-D7 | #13, #14, #15 | spec | Human angiostatin(S) (K1-4) *Afl*III/*Hind*III fragment in pMON5723. (Amino acids 79-440 of plasminogen, nucleotides 346-1431) | This work |
| | | | | |
| pMON24644-E7, -E9, -F8 | ##16, #17, #18 | spec | K2-3 fragment of human angiostatin(S). *Afl*III/*Hind*III fragment in pMON5723. (Amino acids 163-344 of plasminogen, nucleotides 598-1143) | This work |
| | | | | |
| pMON24645-G3, -H5, -H6 | #19, #20, #21 | spec | K2-4 fragment of human angiostatin(S). *Afl*III/*Hind*III fragment in pMON5723. (Amino acids 163-440 of plasminogen, nucleotides 598-1431) | This work |
| | | | | |
| pMON24646-A7, -A9, -A10, -A12, -B1, -B10 | ##22, #23, #24, #25, #26, #27 | spec | K1-3 fragment of human angiostatin(S). *Afl*III/*Hind*III fragment in pMON5723. (Amino acids 79-344 of plasminogen, nucleotides 346-1143) | This work |
| | | | | |
| pMON24649 | #28 | spec | K1 fragment of human angiostatin(S). NcoI/HindIII fragment in pMON5723. (Amino acids 79-184 of plasminogen, nucleotides 1-269 of pMON24646-A7.seq) | This work |
| | | | | |
| pMON24650 | #29 | Amp | K1 fragment of human angiostatin(S). *Nco*I/*Hind*III fragment in pMON3633. (Amino acids 79-184 of plasminogen) | This work |

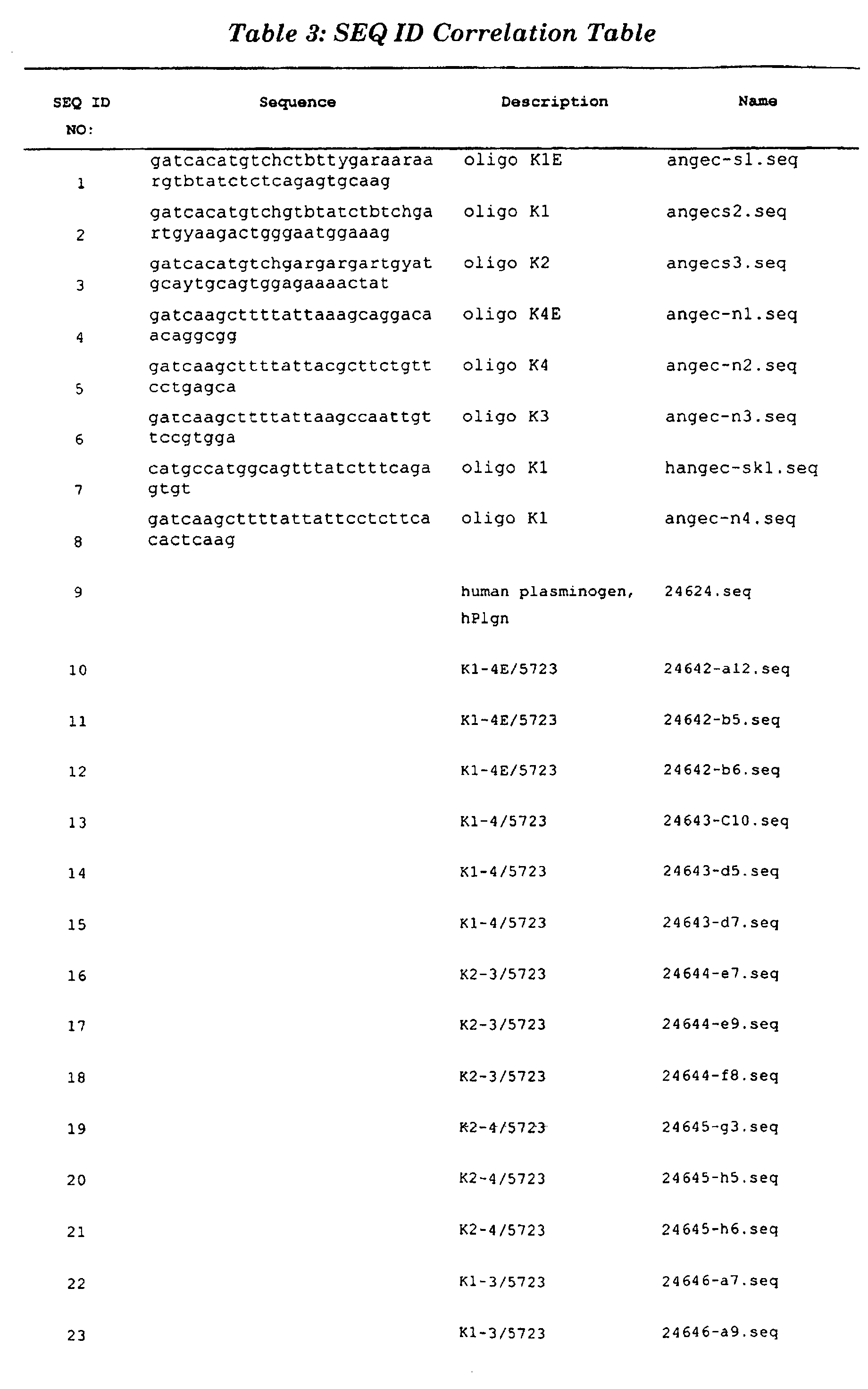

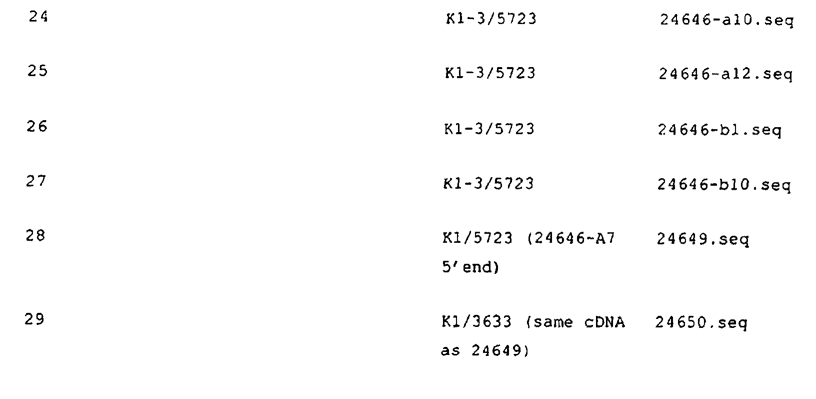

## Claims

1. A method of producing angiostatin comprising the steps of:
(a) culturing host cells that are expressing an angiostatin gene;
(b) recovering the product of said gene expression;
(c) refolding said gene product at an elevated pH from 9 to 11.5; and
(d) isolating properly folded forms of said gene product.

2. A method of producing angiostatin according to claim 1 comprising the steps of:
(a) culturing host cells that are expressing an angiostatin gene;
(b) recovering the product of said gene expression;
(c) solubilizing said gene product at an elevated pH from 9 to 11.5;
(d) refolding said solubilized gene product at an elevated pH from 9 to 11.5; and
(e) isolating properly folded forms of said gene product.

3. The method of claim 1 or 2 wherein said elevated pH is from pH 10 to pH 11.

4. The method of claim 3 wherein said elevated pH is pH 10.5.

5. The method of claim 2 wherein said gene product is present at a concentration of from about 1 to about 20 mg/ml during said solubilization step.

6. The method of claim 5 wherein said gene product is present at a concentration of about 6 mg/ml during said solubilization step.

7. The method of claim 1 or claim 2 wherein said gene product is present at a concentration of from about 0.1 to about 5 mg/m) during said refolding step.

8. The method of claim 7 wherein said gene product is present at a concentration of about 1 mg/ml during said refolding step.

9. The method of claim 1 or claim 2 wherein said angiostatin gene product is encoded by a sequence is selected from the group consisting of SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID N7: 10; SEQ ID NO: 18; SEQ ID NO: 19; SEQ ID NO: 20; SEQ ID NO: 21; SEQ ID NO: 22; SEQ ID NO 23; SEQ ID NO: 24; SEQ ID NO: 25; SEQ ID NO: 26; SEQ ID NO: 27; SEQ ID NO: 28; SEQ ID NO: 29.

10. The method of claim 2 wherein urea is present at a concentration of from about 4 M to about 10 M during said solubilization step.

11. The method of claim 10 wherein urea is present at a concentration of about 6 M during said solubilization step.

12. The method of claim 1 or claim 2 wherein urea is present at a concentration of from about 0.5 M to about 3 M during said refolding step.

13. The method of claim 12 wherein urea is present at a concentration of about 1 M during said refolding step.

14. The method of claim 2 wherein guanidine hydrochloride is present at a concentration of from about 3 M to about 8 M during said solubilization step.

15. The method of claim 14 wherein guanidine hydrochloride is present at a concentration of about 5 M during said solubilization step.

16. The method of claim 1 or claim 2 wherein guanidine hydrochloride is present at a concentration of from about 0.2 M to about 2 M during said refolding step.

17. The method of claim 16 wherein guanidine hydrochloride is present at a concentration of about 0.5 M during said refolding step.

18. A method of claim 1 or 2 carried out in a presence of a reducing agent capable of reducing disulfide linkages to sulfhydryl groups.

19. A method of claim 18 wherein said reducing agent is selected from the group consisting of DTT, BME, cysteine, and reduced glutathione.

20. The method of claim 19 wherein DTT is present at a concentration of from about 2 mM to about 10 mM during said solubilization step.

21. The method of claim 20 wherein DTT is present at a concentration of about 6 mM during said solubilization step.

22. The method of claim 21 wherein DTT is present at a concentration of from about 0.5 mM to about 2 mM during said refolding step.

23. The method of claim 22 wherein DTT is present at a concentration of about 1 mM during said refolding step.

24. The method of claim 19 wherein reduced glutathione is present at a concentration of from about 5 mM to about 20 mM during said solubilization step.

25. The method of claim 24 wherein reduced glutathione is present at a concentration of about 10 mM during said solubilization step.

26. The method of claim 19, wherein reduced glutathione is present at a concentration of from about 1 mM to about 4 mM during said refolding step.

27. The method of claim 26 wherein reduced glutathione is present at a concentration of about 2 mM during said refolding step

28. The method of claim 19 wherein cysteine is present at a concentration of from about 5 mM to about 20 mM during said solubilization step.

29. The method of claim 28 wherein cysteine is present at a concentration of about 10 mM during said solubilization step.

30. The method of claim 19 wherein cysteine is present at a concentration of from about 1 mM to about 4 mM during said refolding step.

31. The method of claim 30 wherein cysteine is present at a concentration of about 2 mM during said refolding step.

32. The method of claim 1 or claim 2 wherein an agent capable of enhancing the interchange of disulfide bonds is present during said refolding step.

33. The method of claim 32 wherein said agent is selected from cystine and oxidized glutathione.

34. The method of claim 33 wherein cystine is present at a concentration of from about 0.2 mM to about 5 mM during said refolding step.

35. The method of claim 34 wherein cystine is present at a concentration of about 1 mM during said refolding step.

36. The method of claim 1 or 2 wherein said refolding step is carried out in the presence of a ligand capable of binding kringle domains of said angiostatin gene product.

37. The method of claim 36 wherein said ligand capable of binding kringle domains of said angiostatin gene product is selected from the group consisting of 6-amino hexanoic acid, 7-aminoheptanoic acid, 5-amino pentanoic acid, and 4-amino butyric acid.

38. The method of claim 37 wherein 6-amino hexanoic acid is present at a concentration of from about 0.2 mM to about 20 mM during said refolding step.

39. The method of claim 38 wherein 6-amino hexanoic acid is present at a concentration of about 1 mM during said refolding step.

40. The method of claim 1 or claim 2 wherein disulfide bonds are formed through air oxidation during said refolding step.

41. The method of claim 40 wherein said air oxidation step is carried out from about 12 hours to about 96 hours.

42. The method of claim 41 wherein said air oxidation step is carried out from about 24 hours to about 72 hours.

43. The method of claim 42 wherein said air oxidation step is carried on for about 60 hours.

44. The method of claim 1 or claim 2 which further includes the step of purifying the angiostatin by a process selected from the group consisting of Lysine-sepharose chromatography, ion-exchange chromatography, hydrophobic interaction chromatography and RP-HPLC.

45. The method of claim 1 or claim 2 wherein said angiostatin gene is comprised of DNA coding for animal angiostatin.

46. The method of claim 1 or claim 2 wherein said angiostatin gene is comprised of DNA coding for human angiostatin.

47. The method of claim 1 or 2 which further includes the step of purifying the angiostatin by a process selected from the group consisting of ion-exchange chromatography, and hydrophobic interaction chromatography.

## Patentansprüche

1. Verfahren zur Herstellung von Angiostatin, umfassend die Stufen:
(a) Kultivieren von Wirtszellen, die ein Angiostatin-Gen exprimieren;
(b) Gewinnen des Produkts der Genexpression;
(c) Rückfaltung des Genprodukts bei einem erhöhten pH von 9 bis 11,5; und
(d) Isolieren richtig gefalteter Formen des Genprodukts.

2. Verfahren zur Herstellung von Angiostatin nach Anspruch 1, umfassend die Stufen:
(a) Kultivieren von Wirtszellen, die ein Angiostatin-Gen exprimieren;
(b) Gewinnen des Produkts der Genexpression;
(c) Solubilisieren des Genprodukts bei einem erhöhten pH von 9 bis 11, 5;
(d) Rückfaltung des solubilisierten Genprodukts bei einem erhöhten pH von 9 bis 11,5;
(e) Isolieren richtig gefalteter Formen des Genprodukts.

3. Verfahren nach Anspruch 1 oder Anspruch 2, wobei der erhöhte pH pH 10 bis pH 11 ist.

4. Verfahren nach Anspruch 3, wobei der erhöhte pH pH 10,5 ist.

5. Verfahren nach Anspruch 2, wobei das Genprodukt während der Solubilisierungsstufe in einer Konzentration von etwa 1 bis etwa 20 mg/ml vorliegt.

6. Verfahren nach Anspruch 5, wobei das Genprodukt während der Solubilisierungsstufe in einer Konzentration von etwa 6 mg/ml vorliegt.

7. Verfahren nach Anspruch 1 oder Anspruch 2, wobei das Genprodukt während der Rückfaltungsstufe in einer Konzentration von etwa 0,1 bis etwa 5 mg/ml vorliegt.

8. Verfahren nach Anspruch 7, wobei das Genprodukt während der Rückfaltungsstufe in einer Konzentration von etwa 1 mg/ml vorliegt.

9. Verfahren nach Anspruch 1 oder Anspruch 2, wobei das Angiostatingenprodukt durch eine Sequenz codiert wird, die aus der Gruppe bestehend aus SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; SEQ ID NO: 18; SEQ ID NO: 19; SEQ ID NO: 20; SEQ ID NO: 21; SEQ ID NO: 22, SEQ ID NO: 23; SEQ ID NO: 24; SEQ ID NO: 25; SEQ ID NO: 26; SEQ ID NO: 27; SEQ ID NO: 28; SEQ ID NO: 29 ausgewählt wird.

10. Verfahren nach Anspruch 2, wobei Harnstoff während der Solubilisierungsstufe in einer Konzentration von etwa 4 M bis etwa 10 M vorhanden ist.

11. Verfahren nach Anspruch 10, wobei Harnstoff in der Solubilisierungsstufe in einer Konzentration von etwa 6 M vorliegt.

12. Verfahren nach Anspruch 1 oder Anspruch 2, wobei Harnstoff während der Rückfaltungsstufe in einer Konzentration von etwa 0,5 M bis etwa 3 M vorliegt.

13. Verfahren nach Anspruch 12, wobei Harnstoff in der Rückfaltungsstufe in einer Konzentration von etwa 1 M vorliegt.

14. Verfahren nach Anspruch 2, wobei Guanidinhydrochlorid während der Solubilisierungsstufe in einer Konzentration von etwa 3 M bis etwa 8 M vorliegt.

15. Verfahren nach Anspruch 14, wobei Guanidinhydrochlorid während der Solubilisierungsstufe in einer Konzentration von etwa 5 M vorliegt.

16. Verfahren nach Anspruch 1 oder Anspruch 2, wobei Guanidinhydrochlorid während der Rückfaltungsstufe in einer Konzentration von etwa 0,2 M bis etwa 2 M vorliegt.

17. Verfahren nach Anspruch 16, wobei Guanidinhydrochlorid während der Rückfaltungsstufe in einer Konzentration von etwa 0,5 M vorliegt.

18. Verfahren nach Anspruch 1 oder 2, das in Gegenwart eines Reduktionsmittels, welches zur Reduktion von Disulfidbindungen zu Sulfhydryl-Gruppen fähig ist, durchgeführt wird.

19. Verfahren nach Anspruch 18, wobei das Reduktionsmittel aus der Gruppe, bestehend aus DTT, BME, Cystein und reduziertem Glutathion, ausgewählt wird.

20. Verfahren nach Anspruch 19, wobei DTT während der Solubilisierungsstufe in einer Konzentration von etwa 2 mM bis etwa 10 mM vorhanden ist.

21. Verfahren nach Anspruch 20, wobei DTT während der Solubilisierungsstufe in einer Konzentration von etwa 6 mM vorliegt.

22. Verfahren nach Anspruch 21, wobei DTT während der Rückfaltungsstufe in einer Konzentration von etwa 0,5 mM bis etwa 2 mM vorliegt.

23. Verfahren nach Anspruch 22, wobei DTT während der Rückfaltungsstufe in einer Konzentration von etwa 1 mM vorliegt.

24. Verfahren nach Anspruch 19, wobei reduziertes Glutathion während der Solubilisierungsstufe in einer Konzentration von etwa 5 mM bis etwa 20 mM vorliegt.

25. Verfahren nach Anspruch 24, wobei reduziertes Glutathion während der Solubilisierungsstufe in einer Konzentration von etwa 10 mM vorliegt.

26. Verfahren nach Anspruch 19, wobei reduziertes Glutathion während der Rückfaltungsstufe in einer Konzentration von etwa 1 mM bis etwa 4 mM vorhanden ist.

27. Verfahren nach Anspruch 26, wobei reduziertes Glutathion während der Rückfaltungsstufe in einer Konzentration von etwa 2 mM vorliegt.

28. Verfahren nach Anspruch 19, wobei Cystein während der Solubilisierungsstufe in einer Konzentration von etwa 5 mM bis etwa 20 mM vorliegt.

29. Verfahren nach Anspruch 28, wobei Cystein während der Solubilisierungsstufe in einer Konzentration von etwa 10 mM vorliegt.

30. Verfahren nach Anspruch 19, wobei Cystein während der Rückfaltungsstufe in einer Konzentration von etwa 1 mM bis etwa 4 mM vorliegt.

31. Verfahren nach Anspruch 30, wobei Cystein während der Rückfaltungsstufe in einer Konzentration von etwa 2 mM vorliegt.

32. Verfahren nach Anspruch 1 oder Anspruch 2, wobei während der Rückfaltungsstufe ein Mittel vorliegt, das fähig ist, den Austausch von Disulfidbindungen zu erhöhen.

33. Verfahren nach Anspruch 32, wobei das Mittel aus Cystin und oxidiertem Glutathion ausgewählt wird.

34. Verfahren nach Anspruch 33, wobei Cystin während der Rückfaltungsstufe in einer Konzentration von etwa 0,2 mM bis etwa 5 mM vorliegt.

35. Verfahren nach Anspruch 34, wobei Cystin während der Rückfaltungsstufe in einer Konzentration von etwa 1 mM vorliegt.

36. Verfahren nach Anspruch 1 oder 2, wobei die Rückfaltungsstufe in Gegenwart eines Liganden durchgeführt wird, der fähig ist, Kringeldomänen des Angiostatingenprodukts zu binden.

37. Verfahren nach Anspruch 36, wobei der Ligand, der fähig ist, Kringeldomänen des Angiostatingenprodukts zu binden, aus der Gruppe bestehend aus 6-Aminohexansäure, 7-Aminoheptansäure, 5-Aminopentansäure und 4-Aminobuttersäure ausgewählt wird.

38. Verfahren nach Anspruch 37, wobei 6-Aminohexansäure während der Rückfaltungsstufe in einer Konzentration von etwa 0,2 mM bis etwa 20 mM vorliegt.

39. Verfahren nach Anspruch 38, wobei 6-Aminohexansäure während der Rückfaltungsstufe in einer Konzentration von etwa 1 mM vorhanden ist.

40. Verfahren nach Anspruch 1 oder Anspruch 2, wobei Disulfidbindungen während der Rückfaltungsstufe durch Luftoxidation gebildet werden.

41. Verfahren nach Anspruch 40, wobei die Stufe der Luftoxidation etwa 12 Stunden bis etwa 96 Stunden durchgeführt wird.

42. Verfahren nach Anspruch 41, wobei die Stufe der Luftoxidation etwa 24 bis etwa 72 Stunden durchgeführt wird.

43. Verfahren nach Anspruch 42, wobei die Stufe der Luftoxidation etwa 60 Stunden durchgeführt wird.

44. Verfahren nach Anspruch 1 oder Anspruch 2, das außerdem die Stufe einer Reinigung des Angiostatins durch ein Verfahren, ausgewählt aus der Gruppe bestehend aus Lysin-Sepharose-Chromatographie, Ionenaustauscher-Chromatographie, hydrophobe Wechselwirkungschromatographie und RP-HPLC, umfasst.

45. Verfahren nach Anspruch 1 oder Anspruch 2, wobei das Angiostatingen von einer DNA umfasst wird, die für Tier-Angiostatin codiert.

46. Verfahren nach Anspruch 1 oder Anspruch 2, wobei das Angiostatingen von einer DNA umfasst wird, die für humanes Angiostatin codiert.

47. Verfahren nach Anspruch 1 oder Anspruch 2, das ferner die Stufe der Reinigung des Angiostatins durch ein Verfahren, ausgewählt aus der Gruppe bestehend aus Ionenaustauscher-Chromatographie und hydrophober Wechselwirkungschromatographie, umfasst.

## Revendications

1. Procédé de production d'angiostatine comprenant les étapes de :
(a) culture de cellules hôtes qui expriment un gène d'angiostatine ;
(b) récupération du produit de l'expression dudit gène ;
(c) repliement dudit produit génique à un pH élevé de 9 à 11,5 ; et
(d) isolement de formes convenablement repliées dudit produit génique.

2. Procédé de production d'angiostatine selon la revendication 1, comprenant les étapes de :
(a) culture de cellules hôtes qui expriment un gène d'angiostatine ;
(b) récupération du produit de l'expression dudit gène ;
(c) solubilisation dudit produit génique à un pH élevé de 9 à 11,5 ;
(d) repliement dudit produit génique solubilisé à un pH élevé de 9 à 11, 5 ; et
(e) isolement de formes convenablement repliées dudit produit génique.

3. Procédé selon la revendication 1 ou 2, où ledit pH élevé est de pH 10 à pH 11.

4. Procédé selon la revendication 3, où ledit pH élevé est pH 10,5.

5. Procédé selon la revendication 2, où ledit produit génique est présent à une concentration d'environ 1 à environ 20 mg/ml pendant ladite étape de solubilisation.

6. Procédé selon la revendication 5, où ledit produit génique est présent à une concentration d'environ 6 mg/ml pendant ladite étape de solubilisation.

7. Procédé selon la revendication 1 ou la revendication 2, où ledit produit génique est présent à une concentration d'environ 0,1 à environ 5 mg/ml pendant ladite étape de repliement.

8. Procédé selon la revendication 7 où ledit produit génique est présent à une concentration d'environ 1 mg/ml pendant ladite étape de repliement.

9. Procédé selon la revendication 1 ou la revendication 2, où ledit produit du gène d'angiostatine est codé par une séquence qui est choisie dans le groupe consistant en SEQ ID NO : 10 ; SEQ ID NO : 11 ; SEQ ID NO : 12 ; SEQ ID NO : 13 ; SEQ ID NO : 14 ; SEQ ID NO : 15 ; SEQ ID NO : 16 ; SEQ ID NO : 17 ; SEQ ID NO : 18 ; SEQ ID NO : 19 ; SEQ ID NO : 20 ; SEQ ID NO : 21 ; SEQ ID NO : 22 ; SEQ ID NO : 23 ; SEQ ID NO : 24 ; SEQ ID NO : 25 ; SEQ ID NO : 26 ; SEQ ID NO : 27 ; SEQ ID NO : 28 ; SEQ ID NO : 29.

10. Procédé selon la revendication 2 où de l'urée est présente à une concentration d'environ 4 M à environ 10 M pendant ladite étape de solubilisation.

11. Procédé selon la revendication 10 où l'urée est présente à une concentration d'environ 6 M pendant ladite étape de solubilisation.

12. Procédé selon la revendication 1 ou la revendication 2 où de l'urée est présente à une concentration d'environ 0,5 M à environ 3 M pendant ladite étape de repliement.

13. Procédé selon la revendication 12 où l'urée est présente à une concentration d'environ 1 M pendant ladite étape de repliement.

14. Procédé selon la revendication 2 où du chlorhydrate de guanidine est présent à une concentration d'environ 3 M à environ 8 M pendant ladite étape de solubilisation.

15. Procédé selon la revendication 14 où le chlorhydrate de guanidine est présent à une concentration d'environ 5 M pendant ladite étape de solubilisation.

16. Procédé selon la revendication 1 ou la revendication 2 où du chlorhydrate de guanidine est présent à une concentration d'environ 0,2 M à environ 2 M pendant ladite étape de repliement.

17. Procédé selon la revendication 16 où le chlorhydrate de guanidine est présent à une concentration d'environ 0,5 M pendant ladite étape de repliement.

18. Procédé selon la revendication 1 ou 2 mis en oeuvre en présence d'un agent réducteur capable de réduire les liaisons disulfure en groupes sulfhydryle.

19. Procédé selon la revendication 18 où ledit agent réducteur est choisi dans le groupe consistant en le DTT, le BME, la cystéine et le glutathion réduit.

20. Procédé selon la revendication 19 où le DTT est présent à une concentration d'environ 2 mM à environ 10 mM pendant ladite étape de solubilisation.

21. Procédé selon la revendication 20 où le DTT est présent à une concentration d'environ 6 mM pendant ladite étape de solubilisation.

22. Procédé selon la revendication 21 où le DTT est présent à une concentration d'environ 0,5 mM à environ 2 mM pendant ladite étape de repliement.

23. Procédé selon la revendication 22 où le DTT est présent à une concentration d'environ 1 mM pendant ladite étape de repliement.

24. Procédé selon la revendication 19 où le glutathion réduit est présent à une concentration d'environ 5 mM à environ 20 mM pendant ladite étape de solubilisation.

25. Procédé selon la revendication 24 où le glutathion réduit est présent à une concentration d'environ 10 mM pendant ladite étape de solubilisation.

26. Procédé selon la revendication 19 où le glutathion réduit est présent à une concentration d'environ 1 mM à environ 4 mM pendant ladite étape de repliement.

27. Procédé selon la revendication 26 où le glutathion réduit est présent à une concentration d'environ 2 mM pendant ladite étape de repliement.

28. Procédé selon la revendication 19 où la cystéine est présente à une concentration d'environ 5 mM à environ 20 mM pendant ladite étape de solubilisation.

29. Procédé selon la revendication 28 où la cystéine est présente à une concentration d'environ 10 mM pendant ladite étape de solubilisation.

30. Procédé selon la revendication 19 où la cystéine est présente à une concentration d'environ 1 mM à environ 4 mM pendant ladite étape de repliement.

31. Procédé selon la revendication 30 où la cystéine est présente à une concentration d'environ 2 mM pendant ladite étape de repliement.

32. Procédé selon la revendication 1 ou la revendication 2 où un agent capable d'augmenter la succession alternée des liaisons disulfure est présent pendant ladite étape de repliement.

33. Procédé selon la revendication 32 où ledit agent est choisi parmi la cystine et le glutathion oxydé.

34. Procédé selon la revendication 33 où la cystine est présente à une concentration d'environ 0,2 mM à environ 5 mM pendant ladite étape de repliement.

35. Procédé selon la revendication 34 où la cystine est présente à une concentration d'environ 1 mM pendant ladite étape de repliement.

36. Procédé selon la revendication 1 ou 2 où ladite étape de repliement est réalisée en présence d'un ligand capable de lier les domaines kringle dudit produit de gène d'angiostatine.

37. Procédé selon la revendication 36 où ledit ligand capable de lier des domaines kringle dudit produit de gène d'angiostatine est choisi dans le groupe consistant en l'acide 6-aminohexanoïque, l'acide 7-aminoheptanoïque, l'acide 5-aminopentanoïque et l'acide 4-aminobutyrique.

38. Procédé selon la revendication 37 où l'acide 6-aminohexanoïque est présent à une concentration d'environ 0,2 mM à environ 20 mM pendant ladite étape de repliement.

39. Procédé selon la revendication 38 où l'acide 6-aminohexanoïque est présent à une concentration d'environ 1 mM pendant ladite étape de repliement.

40. Procédé selon la revendication 1 ou la revendication 2 où des liaisons disulfure sont formées par oxydation à l'air pendant ladite étape de repliement.

41. Procédé selon la revendication 40 où ladite étape d'oxydation à l'air est réalisée pendant environ 12 h à environ 96 h.

42. Procédé selon la revendication 41 où ladite étape d'oxydation à l'air est réalisée pendant environ 24 h à environ 72 h.

43. Procédé selon la revendication 42 où ladite étape d'oxydation à l'air est réalisée pendant environ 60 h.

44. Procédé selon la revendication 1 ou la revendication 2 qui inclut en outre l'étape de purification de l'angiostatine par un procédé choisi dans le groupe consistant en la chromatographie sur lysine-sépharose, la chromatographie d'échange d'ions, la chromatographie d'interaction hydrophobe et la CLHP-PI.

45. Procédé selon la revendication 1 ou la revendication 2 où ledit gène d'angiostatine est constitué par un ADN codant de l'angiostatine animale.

46. Procédé selon la revendication 1 ou la revendication 2 où ledit gène d'angiostatine est constitué par un ADN codant l'angiostatine humaine.

47. Procédé selon la revendication 1 ou 2 qui inclut en outre l'étap de purification de l'angiostatine par un procédé choisi dans le groupe consistant en la chromatographie d'échange d'ions et la chromatographie d'interaction hydrophobe.


### Sequence Listing

### SEQUENCE LISTING

<110> Violand, B.N.
   Polazzi, J.O.
   Casperson, G.F.
<120> Method of refolding of angiostatin
<130> C_3044-0
<150> 60/071,247
   <151> 1998-01-12
<160> 29
<170> FastSEQ for Windows Version 3.0
<210> 1
   <211> 48
   <212> DNA
   <213> human
<400> 1
   gatcacatgt chctbttyga raaraargtb tatctctcag agtgcaag 48
<210> 2
   <211> 48
   <212> DNA
   <213> human
<400> 2
   gatcacatgt chgtbtatct btchgartgy aagactggga atggaaag 48
<210> 3
   <211> 48
   <212> DNA
   <213> human
<400> 3
   gatcacatgt chgargarga rtgyatgcay tgcagtggag aaaactat 48
<210> 4
   <211> 34
   <212> DNA
   <213> human
<400> 4
   gatcaagctt ttattaaagc aggacaacag gcgg 34
<210> 5
   <211> 34
   <212> DNA
   <213> human
<400> 5
   gatcaagctt ttattacgct tctgttcctg agca 34
<210> 6
   <211> 34
   <212> DNA
   <213> human
<400> 6
   gatcaagctt ttattaagcc aattgttccg tgga 34
<210> 7
   <211> 30
   <212> DNA
   <213> human
<400> 7
   catgccatgg cagtttatct ttcagagtgt 30
<210> 8
   <211> 34
   <212> DNA
   <213> human
<400> 8
   gatcaagctt ttattattcc tcttcacact caag 34
<210> 9
   <211> 1719
   <212> DNA
   <213> human
<400> 9
   ggatccgcca ccatggaaca taaggaagtg gttcttctac ttcttttatt tctgaaatca 60
   ggtcaaggag agcctctgga tgactatgtg aatacccagg gggcttcact gttcagtgtc 120
   actaagaagc agctgggagc aggaagtata gaagaatgtg cagcaaaatg tgaggaggac 180
   gaagaattca cctgcagggc attccaatat cacagtaaag agcaacaatg tgtgataatg 240
   gctgaaaaca ggaagtcctc cataatcatt aggatgagag atgtagtttt atttgaaaag 300
   aaagtgtatc tctcagagtg caagactggg aatggaaaga actacagagg gacgatgtcc 360
   aaaacaaaaa atggcatcac ctgtcaaaaa tggagttcca cttctcccca cagacctaga 420
   ttctcacctg ctacacaccc ctcagaggga ctggaggaga actactgcag gaatccagac 480
   aacgatccgc aggggccctg gtgctatact actgatccag aaaagagata tgactactgc 540
   gacattcttg agtgtgaaga ggaatgtatg cattgcagtg gagaaaacta tgacggcaaa 600
   atttccaaga ccatgtctgg actggaatgc caggcctggg actctcagag cccacacgct 660
   catggataca ttccttccaa atttccaaac aagaacctga agaagaatta ctgtcgtaac 720
   cccgataggg agctgcggcc ttggtgtttc accaccgacc ccaacaagcg ctgggaactt 780
   tgtgacatcc cccgctgcac aacacctcca ccatcttctg gtcccaccta ccagtgtctg 840
   aagggaacag gtgaaaacta tcgcgggaat gtggctgtta ccgtgtccgg gcacacctgt     900
   cagcactgga gtgcacagac ccctcacaca cataacagga caccagaaaa cttcccctgc     960
   aaaaatttgg atgaaaacta ctgccgcaat cctgacggaa aaagggcccc atggtgccat    1020
   acaaccaaca gccaagtgcg gtgggagtac tgtaagatac cgtcctgtga ctcctcccca    1080
   gtatccacgg aacaattggc tcccacagca ccacctgagc taacccctgt ggtccaggac    1140
   tgctaccatg gtgatggaca gagctaccga ggcacatcct ccaccaccac cacaggaaag    1200
   aagtgtcagt cttggtcatc tatgacacca caccggcacc agaagacccc agaaaactac    1260
   ccaaatgctg gcctgacaat gaactactgc aggaatccag atgccgataa aggcccctgg    1320
   tgttttacca cagaccccag cgtcaggtgg gagtactgca acctgaaaaa atgctcagga    1380
   acagaagcga gtgttgtagc acctccgcct gttgtcctgc ttccagatgt agagactcct    1440
   tccgaagaag actgtatgtt tgggaatggg aaaggatacc gaggcaagag ggcgaccact    1500
   gttactggga cgccatgcca ggactgggct gcccaggagc cccatagaca cagcattttc    1560
   actccagaga caaatccacg ggcgggtctg gaaaaaaatt actgccgtaa ccctgatggt    1620
   gatgtaggtg gtccctggtg ctacacgaca aatccaagaa aactttacga ctactgtgat    1680
   gtcccwcagt gtgcggcccc ttcatttgat taaggatcc                          1719
<210> 10
   <211> 1154
   <212> DNA
   <213> human
<400> 10
   ccatgtcact ttttgagaag aaggtctatc tctcagagtg caagactggg aatggaaaga      60
   attacagagg gacgatgtcc aaaacaaaaa atggcatcac ctgtcaaaaa tggagttcca     120
   cttctcccca cagacctaga ttctcacctg ctacacaccc ctcagaggga ctggaggaga     180
   actactgcag gaatccagac aacgatccgc aggggccctg gtgctatact actgatccag     240
   aaaagagata tgactactgc gacattcttg agtgtgaaga ggaatgtatg cattgcagtg 300
   gagaaaacta tgacggcaaa atttccaaga ccatgtctgg actggaatgc caggcctggg 360
   actctcagag cccacacgct catggataca ttccttccaa atttccaaac aagaacctga 420
   agaagaatta ctgtcgtaac cccgataggg agctgcggcc ttggtgtttc accaccgacc 480
   ccaacaagcg ctgggaactt tgtgacatcc cccgctgcac aacacctcca ccatcttctg 540
   gtcccaccta ccagtgtctg aagggaacag gtgaaaacta tcgcgggaat gtggctgtta 600
   ccgtgtccgg gcacacctgt cagcactgga gtgcgcagac ccctcacaca cataacagga 660
   caccagaaaa cttcccctgc aaaaatttgg atgaaaacta ctgccgcaat cctgacggaa 720
   aaagggcccc atggtgccat acaaccaaca gccaagtgcg gtgggagtac tgtaagatac 780
   cgtcctgtga ctcctcccca gtatccacgg aacagttggc tcccacagca ccacctgagc 840
   taacccctgt ggtccaggac tgctaccatg gtgatggaca gagctaccga ggcacatcct 900
   ccaccaccac cacaggaaag aagtgtcagt cttggtcatc tatgacacca caccggcacc 960
   agaagacccc agaaaactac ccaaatgctg gcctgacaat gaactactgc aggaatccag 1020
   atgccgataa aggcccctgg tgttttacca cagaccccag cgtcaggtgg gagtactgca 1080
   acctgaaaaa atgctcagga acagaagcga gtgttgtagc acctccgcct gttgtcctgc 1140
   tttaataaaa gctt 1154
<210> 11
   <211> 1154
   <212> DNA
   <213> human
<400> 11
   ccatgtcact gtttgaaaaa aaagtttatc tctcagagtg caagactggg aatggaaaga 60
   attacagagg gacgatgtcc aaaacaaaaa atggcatcac ctgtcaaaaa tggagttcca 120
   cttctcccca cagacctaga ttctcacctg ctacacaccc ctcagaggga ctggaggaga 180
   actactgcag gaatccagac aacgatccgc aggggccctg gtgctatact actgatccag 240
   aaaagagata tgactactgc gacattcttg agtgtgaaga ggaatgtatg cattgcagtg 300
   gagaaaacta tgacggcaaa atttccaaga ccatgtctgg actggaatgc caggcctggg 360
   actctcagag cccacacgct catggataca ttccttccaa atttccaaac aagaacctga 420
   agaagaatta ctgtcgtaac cccgataggg agctgcggcc ttggtgtttc accaccgacc 480
   ccaacaagcg ctgggaactt tgtgacatcc cccgctgcac aacacctcca ccatcttctg 540
   gtcccaccta ccagtgtctg aagggaacag gtgaaaacta tcgcgggaat gtggctgtta 600
   ccgtgtccgg gcacacctgt cagcactgga gtgcacagac ccctcacaca cataacagga 660
   caccagaaaa cttcccctgc aaaaatttgg atgaaaacta ctgccgcaat cctgacggaa 720
   aaagggcccc atggtgccat acaaccaaca gccaagtgcg gtgggagtac tgtaagatac 780
   cgtcctgtga ctcctcccca gtatccacgg aacagttggc tcccacagca ccacctgagc 840
   taacccctgt ggtccaggac tgctaccatg gtgatggaca gagctaccga ggcacatcct 900
   ccaccaccac cacaggaaag aagtgtcagt cttggtcatc tatgacacca caccggcacc 960
   agaagacccc agaaaactac ccaaatgctg gcctgacaat gaactactgc aggaatccag 1020
   atgccgataa aggcccctgg tgttttacca cagaccccag cgtcaggtgg gagtactgca 1080
   acctgaaaaa atgctcagga acagaagcga gtgttgtagc acctccgcct gttgtcctgc 1140
   tttaataaaa gctt 1154
<210> 12
   <211> 1154
   <212> DNA
   <213> human
<400> 12
   ccatgtccct ttttgaaaaa aaagtttatc tctcagagtg caagactggg aatggaaaga 60
   attacagagg gacgatgtcc aaaacaaaaa atggcatcac ctgtcaaaaa tggagttcca 120
   cttctcccca cagacctaga ttctcacctg ctacacaccc ctcagaggga ctggaggaga     180
   actactgcag gaatccagac aacgatccgc aggggccctg gtgctatact actgatccag     240
   aaaagagata tgactactgc gacattcttg agtgtgaaga ggaatgtatg cattgcagtg     300
   gagaaaacta tgacggcaaa atttccaaga ccatgtctgg actggaatgc caggcctggg     360
   actctcagag cccacacgct catggataca ttccttccaa atttccaaac aagaacctga     420
   agaagaatta ctgtcgtaac cccgataggg agctgcggcc ttggtgtttc accaccgacc     480
   ccaacaagcg ctgggaactt tgtgacatcc cccgctgcac aacacctcca ccatcttctg     540
   gtcccaccta ccagtgtctg aagggaacag gtgaaaacta tcgcgggaat gtggctgtta     600
   ccgtgtccgg gcacacctgt cagcactgga gtgcacagac ccctcacaca cataacagga     660
   caccagaaaa cttcccctgc aaaaatttgg atgaaaacta ctgccgcaat cctgacggaa     720
   aaagggcccc atggtgccat acaaccaaca gccaagtgcg gtgggagtac tgtaagatac     780
   cgtcctgtga ctcctcccca gtatccacgg aacagttggc tcccacagca ccacctgagc     840
   taacccctgt ggtccaggac tgctaccatg gtgatggaca gagctaccga ggcacatcct     900
   ccaccaccac cacaggaaag aagtgtcagt cttggtcatc tatgacacca caccggcacc     960
   agaagacccc agaaaactac ccaaatgctg gcctgacaat gaactactgc aggaatccag    1020
   atgccgataa aggcccctgg tgttttacca cagaccccag cgtcaggtgg gagtactgca    1080
   acctgaaaaa atgctcagga acagaagcga gtgttgtagc acctccgcct gttgtcctgc    1140
   tttaataaaa gctt                                                      1154
<210> 13
   <211> 1106
   <212> DNA
   <213> human
<400> 13
   ccatgtcagt gtatctgtcc gagtgtaaga ctgggaatgg aaagaattac agagggacga      60
   tgtccaaaac aaaaaatggc atcacctgtc aaaaatggag ttccacttct ccccacagac      120
   ctagattctc acctgctaca cacccctcag agggactgga ggagaactac tgcaggaatc      180
   cagacaacga tccgcagggg ccctggtgct atactactga tccagaaaag agatatgact      240
   actgcgacat tcttgagtgt gaagaggaat gtatgcattg cagtggagaa aactatgacg      300
   gcaaaatttc caagaccatg tctggactgg aatgccaggc ctgggactct cagagcccac      360
   acgctcatgg atacattcct tccaaatttc caaacaagaa cctgaagaag aattactgtc      420
   gtaaccccga tagggagctg cggccttggt gtttcaccac cgaccccaac aagcgctggg      480
   aactttgtga catcccccgc tgcacaacac ctccaccatc ttctggtccc acctaccagt      540
   gtctgaaggg aacaggtgaa aactatcgcg ggaatgtggc tgttaccgtg tccgggcaca      600
   cctgtcagca ctggagtgca cagacccctc acacacataa caggacacca gaaaacttcc      660
   cctgcaaaaa tttggatgaa aactactgcc gcaatcctga cggaaaaagg gccccatggt      720
   gccatacaac caacagccaa gtgcggtggg agtactgtaa gataccgtcc tgtgactcct      780
   ccccagtatc cacggaacag ttggctccca cagcaccacc tgagctaacc cctgtggtcc      840
   aggactgcta ccatggtgat ggacagagct accgaggcac atcctccacc accaccacag      900
   gaaagaagtg tcagtcttgg tcatctatga caccacaccg gcaccagaag accccagaaa      960
   actacccaaa tgctggcctg acaatgaact actgcaggaa tccagatgcc gataaaggcc     1020
   cctggtgttt taccacagac cccagcgtca ggtgggagta ctgcaacctg aaaaaatgct     1080
   caggaacaga agcgtaataa aagctt                                          1106
<210> 14
   <211> 1106
   <212> DNA
   <213> human
<400> 14
   ccatgtccgt gtacctttct gaatgtaaga ctgggaatgg aaagaattac agagggacga       60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact     240
   actgcgacat tcttgagtgt gaagaggaat gtatgcattg cagtggagaa aactatgacg     300
   gcaaaatttc caagaccatg tctggactgg aatgccaggc ctgggactct cagagcccac     360
   acgctcatgg atacattcct tccaaatttc caaacaagaa cctgaagaag aattactgtc     420
   gtaaccccga tagggagctg cggccttggt gtttcaccac cgaccccaac aagcgctggg     480
   aactttgtga catcccccgc tgcacaacac ctccaccatc ttctggtccc acctaccagt     540
   gtctgaaggg aacaggtgaa aactatcgcg ggaatgtggc tgttaccgtg tccgggcaca     600
   cctgtcagca ctggagtgca cagacccctc acacacataa caggacacca gaaaacttcc     660
   cctgcaaaaa tttggatgaa aactactgcc gcaatcctga cggaaaaagg gccccatggt     720
   gccatacaac caacagccaa gtgcggtggg agtactgtaa gataccgtcc tgtgactcct     780
   ccccagtatc cacggaacag ttggctccca cagcaccacc tgagctaacc cctgtggtcc     840
   aggactgcta ccatggtgat ggacagagct accgaggcac atcctccacc accaccacag     900
   gaaagaagtg tcagtcttgg tcatctatga caccacaccg gcaccagaag accccagaaa     960
   actacccaaa tgctggcctg acaatgaact actgcaggaa tccagatgcc gataaaggcc    1020
   cctggtgttt taccacagac cccagcgtca ggtgggagta ctgcaacctg aaaaaatgct    1080
   caggaacaga agcgtaataa aagctt                                         1106
<210> 15
   <211> 1106
   <212> DNA
   <213> human
<400> 15
   ccatgtccgt ttatctgtct gagtgcaaga ctgggaatgg aaagaattac agagggacga      60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact     240
   actgcgacat tcttgagtgt gaagaggaat gtatgcattg cagtggagaa aactatgacg     300
   gcaaaatttc caagaccatg tctggactgg aatgccaggc ctgggactct cagagcccac     360
   acgctcatgg atacattcct tccaaatttc caaacaagaa cctgaagaag aattactgtc     420
   gtaaccccga tagggagctg cggccttggt gtttcaccac cgaccccaac aagcgctggg     480
   aactttgtga catcccccgc tgcacaacac ctccaccatc ttctggtccc acctaccagt     540
   gtctgaaggg aacaggtgaa aactatcgcg ggaatgtggc tgttaccgtg tccgggcaca     600
   cctgtcagca ctggagtgca cagacccctc acacacataa caggacacca gaaaacttcc     660
   cctgcaaaaa tttggatgaa aactactgcc gcaatcctga cggaaaaagg gccccatggt     720
   gccatacaac caacagccaa gtgcggtggg agtactgtaa gataccgtcc tgtgactcct     780
   ccccagtatc cacggaacag ttggctccca cagcaccacc tgagctaacc cctgtggtcc     840
   aggactgcta ccatggtgat ggacagagct accgaggcac atcctctacc accaccacag     900
   gaaagaagtg tcagtcttgg tcatctatga caccacaccg gcaccagaag accccagaaa     960
   actacccaaa tgctggcctg acaatgaact actgcaggaa tccagatgcc gataaaggcc    1020
   cctggtgttt taccacagac cccagcgtca ggtgggagta ctgcaacctg aaaaaatgct    1080
   caggaacaga agcgtaataa aagctt                                         1106
<210> 16
   <211> 564
   <212> DNA
   <213> human
<400> 16
   ccatgtctga agaagaatgc atgcactgca gtggagaaaa ctatgacggc aaaatttcca     60
   agaccatgtc tggactggaa tgccaggcct gggactctca gagcccacac gctcatggat 120
   acattccttc caaatttcca aacaagaacc tgaagaagaa ttactgtcgt aaccccgata 180
   gggagctgcg gccttggtgt ttcaccaccg accccaacaa gcgctgggaa ctttgtgaca 240
   tcccccgctg cacaacacct ccaccatctt ctggtcccac ctaccagtgt ctgaagggaa 300
   caggtgaaaa ctatcgcggg aatgtggctg ttaccgtgtc cgggcacacc tgtcagcact 360
   ggagtgcaca gacccctcac acacataaca ggacaccaga aaacttcccc tgcaaaaatt 420
   tggatgaaaa ctactgccgc aatcctgacg gaaaaagggc cccatggtgc catacaacca 480
   acagccaagt gcggtgggag tactgtaaga taccgtcctg tgactcctcc ccagtatcca 540
   cggaacaatg gcttataaaa gctt 564
<210> 17
   <211> 564
   <212> DNA
   <213> human
<400> 17
   ccatgtctga ggaagagtgc atgcattgca gtggagaaaa ctatgacggc aaaatttcca 60
   agaccatgtc tggactggaa tgccaggcct gggactctca gagcccacac gctcatgggt 120
   acattccttc caaatttcca aacaagaacc tgaagaagaa ttactgtcgt aaccccgata 180
   gggagctgcg gccttggtgt ttcaccaccg accccaacaa gcgctgggaa ctttgtgaca 240
   ttccccgctg cacaacacct ccaccatctt ctggtcccac ctaccagtgt ctgaagggaa 300
   caggtgaaaa ctatcgcggg aatgtggctg ttaccgtgtc cgggcacacc tgtcagcact 360
   ggagtgcaca gacccctcac acacataaca ggacaccaga aaacttcccc tgcaaaaatt 420
   tggatgaaaa ctactgccgc aatcctgacg gaaaaagggc cccatggtgc catacaacca 480
   acagccaagt gcggtgggag tactgtaaga taccgtcctg tgactcctcc ccagtatcca 540
   cggaacaatg gcttataaaa gctt 564
<210> 18
   <211> 566
   <212> DNA
   <213> human
<400> 18
   mcatgtccga agaagaatgc atgcactgca gtggagaaaa ctatgacggc aaaatttcca      60
   aggccatgtc tggactggaa tgccaggcct gggactctca gagcccacac gctcatggat     120
   acattccttc caaatttcca aacaagaacc tgaagaagaa ttactgtcgt aaccccgata     180
   gggagctgcg gccttggtgt ttcaccaccg accccaacaa gcgctgggaa ctttgtgaca     240
   tcccccgctg cacaacacct ccaccatctt ctggtcccac ctaccagtgt ctgaagggaa     300
   caggtgaaaa ctatcgcggg aatgtggctg ttaccgtgtc cgggcacacc tgtcagcact     360
   ggagtgcaca gacccctcac acacataaca ggacaccaga aaacttcccc tgcaaaaatt     420
   tggatgaaaa ctactgccgc aatcctgacg gaaaaagggc cccatggtgc catacaacca     480
   acagccaagt gcggtgggag tactgtaaga taccgtcctg tgactcctcc ccagtatcca     540
   cggaacaatt ggcttaataa aagctt                                          566
<210> 19
   <211> 854
   <212> DNA
   <213> human
<400> 19
   ccatgtctga ggaagaatgc atgcactgca gtggagaaaa ctatgacggc aaaatttcca      60
   agaccatgtc tggactggaa tgccaggcct gggactctca gagcccacac gctcatggat     120
   acattccttc caaatttcca aacaagaacc tgaagaagaa ttactgtcgt aaccccgata 180
   gggagctgcg gccttggtgt ttcaccaccg accccaacaa gcgctgggaa ctttgtgaca 240
   tcccccgctg cacaacacct ccaccatctt ctggtcccac ctaccagtgt ctgaagggaa 300
   caggtgaaaa ctatcgcggg aatgtggctg ttaccgtgtc cgggcacacc tgtcagcact 360
   ggagtgcaca gacccctcac acacataaca ggacaccaga aaacttcccc tgcaaaaatt 420
   tggatgaaaa ctactgccgc aatcctgacg gaaaaagggc cccatggtgc catacaacca 480
   acagccaagt gcggtgggag tactgtaaga taccgtcctg tgactcctcc ccagtatcca 540
   cggaacagtt ggctcccaca gcaccacctg agctaacccc tgtggtccag gactgctacc 600
   atggtgatgg acagagctac cgaggcacat cctccaccac caccacagga aagaagtgtc 660
   agtcttggtc atctatgaca ccacaccggc accagaagac cccagaaaac tacccaaatg 720
   ctggcctgac aatgaactac tgcaggaatc cagatgccga taaaggcccc tggtgtttta 780
   ccacagaccc cagcgtcagg tgggagtact gcaacctgaa aaaatgctca ggaacagaag 840
   cgtaataaaa gctt 854
<210> 20
   <211> 854
   <212> DNA
   <213> human
<400> 20
   ccatgtcaga ggaagagtgc atgcactgca gtggagaaaa ctatgacggc aaaatttcca 60
   agaccatgtc tggactggaa tgccaggcct gggactctca gagcccacac gctcatggat 120
   acattccttc caaatttcca aacaagaacc tgaagaagaa ttactgtcgt aaccccgata 180
   gggagctgcg gccttggtgt ttcaccaccg accccaacaa gcgctgggaa ctttgtgaca 240
   tcccccgctg cacaacacct ccaccatctt ctggtcccac ctaccagtgt ctgaagggaa 300
   caggtgaaaa ctatcgcggg aatgtggctg ttaccgtgtc cgggcacacc tgtcagcact 360
   ggagtgcaca gacccctcac acacataaca ggacaccaga aaacttcccc tgcaaaaatt     420
   tggatgaaaa ctactgccgc aatcctgacg gaaaaagggc cccatggtgc catacaacca     480
   acagccaagt gcggtgggag tactgtaaga taccgtcctg tgactcctcc ccagtatcca     540
   cggaacagtt ggctcccaca gcaccacctg agctaacccc tgtggtccag gactgctacc     600
   atggtgatgg acagagctgc cgaggcacat cctccaccac caccacagga aagaagtgtc     660
   agtcttggtc atctatgaca ccacaccggc accagaagac cccagaaaac tacccaaatg     720
   ctggcctgac aatgaactac tgcaggaatc cagatgccga taaaggcccc tggtgtttta     780
   ccacagaccc cagcgtcagg tgggagtact gcaacctgaa aaaatgctca ggaacagaag     840
   cgtaataaaa gctt                                                       854
<210> 21
   <211> 854
   <212> DNA
   <213> human
<400> 21
   ccatgtccga agaagagtgc atgcactgca gtggagaaaa ctatgacggc aaaatttcca      60
   agaccatgtc tggactggaa tgccaggcct gggactctca gagcccacac gctcatggat     120
   acattccttc caaatttcca aacaagaacc tgaagaagaa ttactgtcgt aaccccgata     180
   gggagctgcg gccttggtgt ttcaccaccg accccaacaa gcgctgggaa ctttgtgaca     240
   tcccccgctg cacaacacct ccaccatctt ctggtcccac ctaccagtgt ctgaagggaa     300
   caggtgaaaa ctatcgcggg aatgtggctg ttaccgtgtc cgggcacacc tgtcagcact     360
   ggagtgcaca gacccctcac acacataaca ggacaccaga aaacttcccc tgcaaaaatt     420
   tggatgaaaa ctactgccgc aatcctgacg gaaaaagggc cccatggtgc catacaacca     480
   acagccaagt gcggtgggag tactgtaaga taccgtcctg tgactcctcc ccagtatcca     540
   cggaacagtt ggctcccaca gcaccacctg agctaacccc tgtggtccag gactgctacc     600
   atggtgatgg acagagctac cgaggcacat cctccaccac caccacagga aagaagtgtc 660
   agtcttggtc atctatgaca ccacaccggc accagaagac cccagaaaac tacccaaatg 720
   ctggcctgac aatgaactac tgcaggaatc cagatgccga taaaggcccc tggtgtttta 780
   ccacagaccc cagcgtcagg tgggagtact gcaacctgaa aaaatgctca ggaacagaag 840
   cgtaataaaa gctt 854
<210> 22
   <211> 818
   <212> DNA
   <213> human
<400> 22
   ccatgtcagt ttatctttca gagtgtaaga ctgggaatgg aaagaattac agagggacga 60
   tgtccaaaac aaaaaatggc atcacctgtc aaaaatggag ttccacttct ccccacagac 120
   ctagattctc acctgctaca cacccctcag agggactgga ggagaactac tgcaggaatc 180
   cagacaacga tccgcagggg ccctggtgct atactactga tccagaaaag agatatgact 240
   actgcgacat tcttgagtgt gaagaggaat gtatgcattg cagtggagaa aactatgacg 300
   gcaaaatttc caagaccatg tctggactgg aatgccaggc ctgggactct cagagcccac 360
   acgctcatgg atacattcct tccaaatttc caaacaagaa cctgaagaag aattactgtc 420
   gtaaccccga tagggagctg cggccttggt gtttcaccac cgaccccaac aagcgctggg 480
   aactttgtga catcccccgc tgcacaacac ctccaccatc ttctggtccc acctaccagt 540
   gtctgaaggg aacaggtgaa aactatcgcg ggaatgtggc tgttaccgtg tccgggcaca 600
   cctgtcagca ctggagtgca cagacccctc acacacataa caggacacca gaaaacttcc 660
   cctgcaaaaa tttggatgaa aactactgcc gcaatcctga cggaaaaagg gccccatggt 720
   gccatacaac caacagccaa gtgcggtggg agtactgtaa gataccgtcc tgtgactcct 780
   ccccagtatc cacggaacaa ttggcttaat aaaagctt 818
<210> 23
   <211> 818
   <212> DNA
   <213> human
<400> 23
   ccatgtcagt gtacctttct gaatgtaaga ctgggaatgg aaagaattac agagggacga      60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact     240
   actgcgacat tcttgagtgt gaagaggaat gtatgcattg cagtggagaa aactatgacg     300
   gcaaaatttc caagaccatg tctggactgg aatgccaggc ctgggactct cagagcccac     360
   acgctcatgg atacattcct tccaaatttc caaacaagaa cctgaagaag aattactgtc     420
   gtaaccccga tagggagctg cggccttggt gtttcaccac cgaccccaac aagcgctggg     480
   aactttgtga catcccccgc tgcacaacac ctccaccatc ttctggtccc acctaccagt     540
   gtctgaaggg aacaggtgaa aactatcgcg ggaatgtggc tgttaccgtg tccgggcaca     600
   cctgtcagca ctggagtgca cagacccctc acacacataa caggacacca gaaaacttcc     660
   cctgcaaaaa tttggatgaa aactactgcc gcaatcctga cggaaaaagg gccccatggt     720
   gccatacaac caacagccaa gtgcggtggg agtactgtaa gataccgtcc tgtgactcct     780
   ccccagtatc cacggaacaa ttggcttaat aaaagctt                             818
<210> 24
   <211> 818
   <212> DNA
   <213> human
<400> 24
   ccatgtcagt ttatctttca gagtgtaaga ctgggaatgg aaagaattac agagggacga      60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact     240
   actgcgacat tcttgagtgt gaagaggaat gtatgcattg cagtggagaa aactatgacg     300
   gcaaaatttc caagaccatg tctggactgg aatgccaggc ctgggactct cagagcccac     360
   acgctcatgg atacattcct tccaaatttc caaacaagaa cctgaagaag aattactgtc     420
   gtaaccccga tagggagctg cggccttggt gtttcaccac cgaccccaac aagcgctggg     480
   aactttgtga catcccccgc tgcacaacac ctccaccatc ttctggtccc acctaccagt     540
   gtctgaaggg aacaggtgaa aactatcgcg ggaatgtggc tgttaccgtg tccgggcaca     600
   cctgtcagca ctggagtgca cagacccctc acacacataa caggacacca gaaaacttcc     660
   cctgcaaaaa tttggatgaa aactactgcc gcaatcctga cggaaaaagg gccccatggt     720
   gccatacaac caacagccaa gtgcggtggg agtactgtaa gataccgtcc tgtgactcct     780
   ccccagtatc cacggaacaa ttggcttaat aaaagctt                             818
<210> 25
   <211> 818
   <212> DNA
   <213> human
<400> 25
   ccatgtctgt ttacctgtct gaatgtaaga ctgggaatgg aaagaattac agagggacga      60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact 240
   actgcgacat tcttgagtgt gaagaggaat gtatgcattg cagtggagaa aactatgacg 300
   gcaaaatttc caagaccatg tctggactgg aatgccaggc ctgggactct cagagcccac 360
   acgctcatgg atacattcct tccaaatttc caaacaagaa cctgaagaag aattactgtc 420
   gtaaccccga tagggagctg cggccttggt gtttcaccac cgaccccaac aagcgctggg 480
   aactttgtga catcccccgc tgcacaacac ctccaccatc ttctggtccc acctaccagt 540
   gtctgaaggg aacaggtgaa aactatcgcg ggaatgtggc tgttaccgtg tccgggcaca 600
   cctgtcagca ctggagtgca cagacccctc acacacataa caggacacca gaaaacttcc 660
   cctgcaaaaa tttggatgaa aactactgcc gcaatcctga cggaaaaagg gccccatggt 720
   gccatacaac caacagccaa gtgcggtggg agtactgtaa gataccgtcc tgtgactcct 780
   ccccagtatc cacggaacaa ttggcttaat aaaagctt 818
<210> 26
   <211> 818
   <212> DNA
   <213> human
<400> 26
   ccatgtccgt ttatctttcc gagtgcaaga ctgggaatgg aaagaattac agagggacga 60
   tgtccaaaac aaaaaatggc atcacctgtc aaaaatggag ttccacttct ccccacagac 120
   ctagattctc acctgctaca cacccctcag agggactgga ggagaactac tgcaggaatc 180
   cagacaacga tccgcagggg ccctggtgct atactactga tccagaaaag agatatgact 240
   actgcgacat tcttgagtgt gaagaggaat gtatgcattg cagtggagaa aactatgacg 300
   gcaaaatttc caagaccatg tctggactgg aatgccaggc ctgggactct cagagcccac 360
   acgctcatgg atacattcct tccaaatttc caaacaagaa cctgaagaag aattactgtc 420
   gtaaccccga tagggagctg cggccttggt gtttcaccac cgaccccaac aagcgctggg 480
   aactttgtga catcccccgc tgcacaacac ctccaccatc ttctggtccc acctaccagt 540
   gtctgaaggg aacaggtgaa aactatcgcg ggaatgtggc tgttaccgtg tccgggcaca 600
   cctgtcagca ctggagtgca cagacccctc acacacataa caggacacca gaaaacttcc 660
   cctgcaaaaa tttggatgaa aactactgcc gcaatcctga cggaaaaagg gccccatggt 720
   gccatacaac caacagccaa gtgcggtggg agtactgtaa gataccgtcc tgtgactcct 780
   ccccagtatc cacggaacaa ttggcttaat aaaagctt 818
<210> 27
   <211> 818
   <212> DNA
   <213> human
<400> 27
   ccatgtctgt gtacctgtcc gaatgtaaga ctgggaatgg aaagaattac agagggacga 60
   tgtccaaaac aaaaaatggc atcacctgtc aaaaatggag ttccacttct ccccacagac 120
   ctagattctc acctgctaca cacccctcag agggactgga ggagaactac tgcaggaatc 180
   cagacaacga tccgcagggg ccctggtgct atactactga tccagaaaag agatatgact 240
   actgcgacat tcttgagtgt gaagaggaat gtatgcattg cagtggagaa aactatgacg 300
   gcaaaatttc caagaccatg tctggactgg aatgccaggc ctgggactct cagagcccac 360
   acgctcatgg atacattcct tccaaatttc caaacaagaa cctgaagaag aattactgtc 420
   gtaaccccga tagggagctg cggccttggt gtttcaccac cgaccccaac aagcgctggg 480
   aactttgtga catcccccgc tgcacaacac ctccaccatc ttctggtccc acctaccagt 540
   gtctgaaggg aacaggtgaa aactatcgcg ggaatgtggc tgttaccgtg tccgggcaca 600
   cctgtcagca ctggagtgca cagacccctc acacacataa caggacacca gaaaacttcc 660
   cctgcaaaaa tttggatgaa aactactgcc gcaatcctga cggaaaaagg gccccatggt 720
   gccatacaac caacagccaa gtgcggtggg agtactgtaa gataccgtcc tgtgactcct 780
   ccccagtatc cacggaacaa ttggcttaat aaaagctt 818
<210> 28
   <211> 281
   <212> DNA
   <213> human
<400> 28
   ccatggcagt ttatctttca gagtgtaaga ctgggaatgg aaagaattac agagggacga      60
   tgtccaaaac aaaaaatggc atcacctgtc aaaaatggag ttccacttct ccccacagac     120
   ctagattctc acctgctaca cacccctcag agggactgga ggagaactac tgcaggaatc     180
   cagacaacga tccgcagggg ccctggtgct atactactga tccagaaaag agatatgact     240
   actgcgacat tcttgagtgt gaagaggaat aataaaagct t                         281
<210> 29
   <211> 345
   <212> DNA
   <213> human
<400> 29
   ggatccacca tgagccgcct gcccgtcctg ctcctgctcc aactcctggt ccgccccgcc      60
   atggccgtgt acctgtcaga atgtaagact gggaatggaa agaattacag agggacgatg     120
   tccaaaacaa aaaatggcat cacctgtcaa aaatggagtt ccacttctcc ccacagacct     180
   agattctcac ctgctacaca cccctcagag ggactggagg agaactactg caggaatcca     240
   gacaacgatc cgcaggggcc ctggtgctat actactgatc cagaaaagag atatgactac     300
   tgcgacattc ttgagtgtga agaggaataa taaaagcttg gattc                     345